# EUROPEAN PATENT APPLICATION

(11) **EP 3 653 642 A1**
(43) Date of publication of application: **20.05.2020**
(21) Application number: 19198850.0
(22) Date of filing: 17.03.2014
(51) Int. Cl.: C07K 16/18, C07K 16/28, C07K 16/30, C07K 16/46, A61P 35/00

(54) **SINGLE CHAIN BINDING MOLECULES COMPRISING N-TERMINAL ABP**

(30) Priority: 15.03.2013 US 201361792073 P
(62) Divisional of application: 14718334.7
(71) Applicant: Amgen Research (Munich) GmbH, 81477 München (DE)
(72) Inventor: Kufer, Peter, 81477 Munich (DE); Lutterbuese, Ralf, 81477 Munich (DE); Hoffmann, Patrick, 81477 Munich (DE); Nahrwold, Elisabeth, 81477 Munich (DE)
(74) Representative: Schiweck Weinzierl Koch Patentanwälte Partnerschaft mbB

(57) **Abstract**

The present invention relates to a single chain binding molecule comprising at least three binding domains, wherein the first binding domain is capable of binding to serum albumin and is positioned at the N-terminus of the second binding domain, said second binding domain is capable of binding to a cell surface molecule on a target cell and the third binding domain is capable of binding to the T cell CD3 receptor complex. Moreover, the invention relates to methods for the production of such binding molecule, a nucleic acid sequence encoding it, a vector comprising said nucleic acid sequence and a host cell expressing the binding molecule of the invention. Furthermore, the invention relates to a pharmaceutical composition comprising a binding molecule of the invention, methods of treatment comprising the step of administering a binding molecule of the invention and the medical use of a binding molecule of the invention.

## Description

### Field of the Invention

The present invention provides a single chain binding molecule comprising at least three binding domains, wherein the first binding domain is capable of binding to serum albumin and is positioned at the N-terminus of the second binding domain, said second binding domain is capable of binding to a cell surface molecule on a target cell and the third binding domain is capable of binding to the T cell CD3 receptor complex. Moreover, the invention provides a methods for the production of such binding molecule, a nucleic acid sequence encoding it, a vector comprising said nucleic acid sequence and a host cell expressing the binding molecule of the invention. Furthermore, the invention provides a pharmaceutical composition comprising a binding molecule of the invention, methods of treatment comprising the step of administering a binding molecule of the invention and the medical use of a binding molecule of the invention.

### Background of the invention

An increased half-life is generally useful in in vivo applications of immunoglobulins, especially antibodies and most especially antibody fragments of small size. Such fragments (Fvs, disulphide bonded Fvs, Fabs, scFvs, dAbs) are likely to suffer from rapid clearance from the body; thus, whilst they are able to reach most parts of the body rapidly, and are quick to produce and easier to handle, their in vivo applications may be limited by their brief persistence in vivo.

Bispecific molecules such as BiTE (Bispecific T-cell engager) antibodies are recombinant protein constructs made from two flexibly linked, single-chain antibodies (scFv). One scFv of BiTE antibodies is specific for a selected tumor-associated surface antigen on target cells; the second scFv is specific for CD3, a subunit of the T-cell receptor complex on T-cells. By their particular design and bivalent binding, BiTE antibodies are uniquely suited to transiently bind T-cells to target cells and, at the same time, potently activate the inherent cytolytic potential of T-cells against target cells. BiTE molecules are small proteins with a molecular weight below the renal cut off that could likely result in a shorter half-life, a feature that is shared by BiTEs with many other antibody formats. In fact, while it is one the one hand desirable to have a small binding molecule, since, for example, it can quickly reach its designated location in the body and can also reach most parts of the body, the "size" of such a binding molecule is not favorable as regards, in particular, renal clearance. It may also happen that such a binding molecule is faster degraded, since it has, so to say, no good natural protection, unless it was stabilized before, for example, by amino acid changes. Thus, it is a balancing act between small size and stability/renal clearance.

It is therefore desirable to have available a binding molecule, in particular, a bispecific binding molecule that is improved in its stability and/or retarded in its renal clearance, thereby having an overall increased serum half-life, but advantageously still so small that it can be manufactured in good yield and/or conveniently handled.

### Summary of the invention

The present invention provides a single chain binding molecule comprising at least three binding domains, wherein
(a) the first binding domain is capable of binding to serum albumin and is positioned at the N-terminus of the second binding domain;
(b) said second binding domain is capable of binding to a cell surface molecule on a target cell; and
(c) the third binding domain is capable of binding to the T cell CD3 receptor complex.

In one embodiment the binding molecule of the invention is characterized in a way that the three domains are consecutively on one polypeptide chain in the order from the N-terminus to the C-terminus
- the first binding domain;
- the second binding domain; and
- the third binding domain.

The invention also provides a single chain binding molecule comprising at least three binding domains comprised in one polypeptide chain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex,
wherein the percentage of expressible monomeric binding molecule [in relation to the total amount of binding molecule] depends on the order of the first and second binding domain in said binding molecule.

The invention further provides a single chain binding molecule comprising at least three binding domains comprised in one polypeptide chain in the order first domain, second domain and third domain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex;
wherein the yield of monomeric binding molecule isolated from the culture supernatant of host cells producing the binding molecule is at least 1.5 times higher than the yield of monomeric binding molecule isolated from the culture supernatant of host cells producing a binding molecule comprising the binding domain capable of binding to serum albumin is at the C-terminus of the molecule.

In one embodiment the binding molecule of the invention is characterized in a way that at least one of the binding domains, preferably the second and/or third binding domain, is an scFv or single domain antibody.

Also in one embodiment of the binding molecule of the invention the molecule comprises one or more further heterologous polypeptide.

A binding molecule of the invention may also comprise a His-tag as a heterologus poypeptide. It is preferred for the binding molecule of the invention that the His-tag is positioned at the C-terminus of the third binding domain.

The invention also provides a binding molecule, wherein
(a) the first binding domain is capable of binding to human and non-human primate serum albumin;
(b) the second binding domain is capable of binding to the cell surface molecule on a human and a non-human primate cell, and
(c) the third binding domain is capable of binding to the T cell CD3 receptor complex on a human and a non-human primate cell.

In one embodiment the binding molecule according to the invention is characterized that the first binding domain capable of binding to serum albumin is derived from a combinatorial library or an antibody binding domain.

In a preferred embodiment of the binding molecule of the invention the first binding domain comprises between 10 and 25 aa residues.

In one embodiment of the binding molecule of the invention the first binding domain capable of binding to serum albumin comprises the amino acid sequence Asp-Xaa-Cys-Leu-Pro-Xaa-Trp-Gly-Cys-Leu-Trp, wherein Xaa is any amino acid.

In one embodiment of the binding molecule of the invention the first binding domain capable of binding to serum albumin is derived from a CDR of a single domain antibody.

Also in one embodiment of the binding molecule of the invention the first binding domain is binding to serum albumin with an affinity (KD) of ≤ 500 nM.

In one embodiment of the binding molecule of the invention
- the second binding domain is binding to the cell surface molecule on a target cell with an affinity (KD) of ≤ 100 nM; and
- the third binding domain is binding to the T cell CD3 receptor complex with an affinity (KD) of ≤ 100nM.

In one embodiment of the binding molecule of the invention the binding molecule shows cytotoxic activity in an in vitro assay measuring the lysis of target cells by effector cells in the presence of 10% human serum albumin.

In a preferred embodiment of the binding molecule of the invention the molecule consists of a single polypeptide chain.

In one embodiment of the binding molecule of the invention
(a) the second binding domain comprises an antibody derived VL and VH chain; and/or
(b) the second binding domain comprises an antibody derived VL and VH chain.

In one embodiment of the binding molecule of the invention the first binding domain capable of binding to a cell surface molecule is binding to a tumor antigen.

In a preferred embodiment of the binding molecule of the invention the second binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 of WO 2008/119567 and comprises at least the amino acid sequence Gln-Asp-Gly-Asn-Glu (SEQ ID NO:37).

In a preferred embodiment of the invention the second binding domain is capable of binding CD33 or CEA.

In one embodiment the binding molecule of the invention is characterized by an amino acid sequence as depicted in SEQ ID NOs: 8, 12, 16, 20, 24, 26, 30, or 34.

An alternative embodiment of the invention provides a method for the production of binding molecule of the invention, the method comprising the step of:
- selecting for binding molecules comprising a binding domain, which is capable of binding to a cell surface molecule on a target cell, comprising at the N-terminus a binding domain which is capable of binding to serum albumin.

In one embodiment the method of the invention further comprises the step of:
- adding to the molecule an additional binding domain, which is capable of binding to the T cell CD3 receptor complex,.

With one embodiment the invention provides a nucleic acid molecule having a sequence encoding a binding molecule of the invention.

The invention also provides a vector comprising a nucleic acid sequence of the invention.

The invention provides host cell transformed or transfected with the nucleic acid s of the invention or with the vector of the invention.

In one embodiment the invention provides a process for the production of a binding molecule of the invention or produced by a method of the invention, said process comprising culturing a host cell of the invention under conditions allowing the expression of the binding molecule a of the invention or produced by a method of the invention and recovering the produced binding molecule from the culture.

The invention also provides a pharmaceutical composition comprising a binding molecule of the invention, or produced according to the process of the invention.

According to one embodiment of the invention the binding molecule of the invention, or the binding molecule produced according to a method of the invention is for use in the prevention, treatment or amelioration of a disease selected from the group consisting of a proliferative disease, an inflammatory disease, an infectious disease and an autoimmune disease.

In one embodiment the invention provides a method for the treatment or amelioration of a disease selected from the group consisting of a proliferative disease, an inflammatory disease, an infectious disease and an autoimmune disease, comprising the step of administering to a subject in need thereof the binding molecule of the invention, or the binding molecule produced according to a method of the invention.

Also in one embodiment the invention provides a kit comprising a binding molecule of the invention or produced according to a method of the invention, a nucleic acid molecule of the invention, a vector of the invention, or a host cell of the invention.

### Brief description of the drawings

**Figure 1****:** Graph of an elution profile from the IMAC purification of a SA-21-CEAxCD3 binding molecule. 1: Capture, 2: Wash out unbound sample, 3: Pre-Elution 50 mM Imidazole, 4: Elution 500 mM Imidazole. Blue line: Optical absorption at 280 nm, Red line: Optical absorption at 254 nm.
**Figure 2****:** Graph of an SEC profile for a SA-21-CEAxCD3 binding molecule. Peak 1: Aggregates (Non binding molecules) in void volume. Peak 2: binding molecule dimer. Peak 3: binding molecule monomer. Peak 4: Low molecular weight contaminants and salts. Optical absorption at 280 nm, Red line: Optical absorption at 254 nm.
**Figure 3****:** Cytotoxic activity of CD33 bispecific antibodies as measured in an 18-hour ⁵¹chromium release assay in presence of 10% HSA. Effector cells: stimulated enriched human CD8 T cells. Target cells: Human CD33 transfected CHO cells. Effector to target cell (E:T) ratio: 10:1.

### Detailed description of the invention

### Definitions:

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within ±20%, preferably within ±15%, more preferably within ±10%, and most preferably within ±5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

The term "binding molecule" or "antibody construct" in the sense of the present disclosure indicates any molecule capable of (specifically) binding to, interacting with or recognizing the target molecule. For the second binding domain such target molecule is a cell surface molecule on a target cell. For the third binding domain such target molecule is the T cell CD3 receptor complex.
The term "single chain binding molecule" defines in connection with the present invention that the disclosed binding molecules in its simplest form are monomers. The molecules or constructs may include proteinaceous parts and non-proteinaceous parts (e.g. chemical linkers or chemical cross-linking agents such as glutaraldehyde). Thus, the single chain binding molecule may comprising accordance with the invention non-peptide linkers preferably to link at least two of the binding domains. Also in line with this invention are herein defined peptide linkers.

A binding molecule, so to say, provides the scaffold for said one or more binding domains so that said binding domains can bind/interact with the surface molecule on a target cell and CD3 receptor complex on a T cell. For example, such a scaffold could be provided by protein A, in particular, the Z-domain thereof (affibodies), ImmE7 (immunity proteins), BPTI/APPI (Kunitz domains), Ras-binding protein AF-6 (PDZ-domains), charybdotoxin (Scorpion toxin), CTLA-4, Min-23 (knottins), lipocalins (anticalins), neokarzinostatin, a fibronectin domain, an ankyrin consensus repeat domain (Stumpp et al., Curr Opin Drug Discov Devel. 10(2), 153-159 (2007)) or thioredoxin (Skerra, Curr. Opin. Biotechnol. 18, 295-304 (2005); Hosse et al., Protein Sci. 15, 14-27 (2006); Nicaise et al., Protein Sci. 13, 1882-1891 (2004); Nygren and Uhlen, Curr. Opin. Struc. Biol. 7, 463-469 (1997)). A preferred binding molecule is an antibody, more preferably a bispecific antibody.

The definition of the term "antibody" includes embodiments such as monoclonal, chimeric, single chain, humanized and human antibodies, as well as antibody fragments, like, inter alia, Fab fragments. Antibody fragments or derivatives further comprise F(ab')₂, Fv, scFv fragments or single domain antibodies such as domain antibodies or nanobodies, single variable domain antibodies or immunoglobulin single variable domain comprising merely one variable domain, which might be VHH, VH or VL, that specifically bind an antigen or epitope independently of other V regions or domains; see, for example, Harlow and Lane (1988) and (1999), loc. cit.; Kontermann and Dübel, Antibody Engineering, Springer, 2nd ed. 2010 and Little, Recombinant Antibodies for Immunotherapy, Cambridge University Press 2009. Such immunoglobulin single variable domain encompasses not only an isolated antibody single variable domain polypeptide, but also larger polypeptides that comprise one or more monomers of an antibody single variable domain polypeptide sequence.
Monovalent antibody fragments in line with the above definition describe an embodiment of a binding domain in connection with this invention. Such monovalent antibody fragments bind to a specific antigen and can be also designated "antigen-binding domain", "antigen-binding fragment" or "antibody binding region".
In line with this definition all above described embodiments of the term antibody can be subsumed under the term "antibody construct". Said term also includes diabodies or Dual-Affinity Re-Targeting (DART) antibodies. Further envisaged are (bispecific) single chain diabodies, tandem diabodies (Tandab's), "minibodies" exemplified by a structure which is as follows: (VH-VL-CH3)₂, (scFv-CH3)₂ or (scFv-CH3-scFv)₂, "Fc DART" antibodies and "IgG DART" antibodies, and multibodies such as triabodies. Immunoglobulin single variable domains encompass not only an isolated antibody single variable domain polypeptide, but also larger polypeptides that comprise one or more monomers of an antibody single variable domain polypeptide sequence.
Various procedures are known in the art and may be used for the production of such antibody constructs (antibodies and/or fragments). Thus, (antibody) derivatives can be produced by peptidomimetics. Further, techniques described for the production of single chain antibodies (see, inter alia, US Patent 4,946,778, Kontermann and Dübel (2010), loc. cit. and Little(2009), loc. cit.) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Kohler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.
Furthermore, the term "antibody" as employed in the invention also relates to derivatives or variants of the antibodies described herein which display the same specificity as the described antibodies.
The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv); see e.g., Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U. S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The term "human antibody" includes antibodies having variable and constant regions corresponding substantially to human germline immunoglobulin sequences known in the art, including, for example, those described by Kabat *et al.* (See Kabat *et al.* (1991) loc. cit.). The human antibodies of the invention may include amino acid residues not encoded by human germline immunoglobulin sequences (e.g., mutations introduced by random or site-specific mutagenesis *in vitro* or by somatic mutation *in vivo),* for example in the CDRs, and in particular, CDR3. The human antibody can have at least one, two, three, four, five, or more positions replaced with an amino acid residue that is not encoded by the human germline immunoglobulin sequence. It is emphasized that the definition of human antibodies as used herein also contemplates fully human antibodies, which include only non-artificially and/or genetically altered human sequences of antibodies as those can be derived by using technologies using systems such as the Xenomice.
Examples of "antibody variants" include humanized variants of non- human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260, Kontermann and Dübel (2010), loc. cit. and Little(2009), loc. cit.).

As used herein, *"in vitro* generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of four regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3.
The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (i.e., non-hypervariable) portions of the variable domains are called the "framework" regions (FRM). The variable domains of naturally occurring heavy and light chains each comprise four FRM regions, largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat *et al.,* loc. cit.). The constant domains are not directly involved in antigen binding, but exhibit various effector functions, such as, for example, antibody-dependent, cell-mediated cytotoxicity and complement activation.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum (Kabat *et al.,* loc. cit.; Chothia et al., J. Mol. Biol, 1987, 196: 901; and MacCallum et al., J. Mol. Biol, 1996, 262: 732). However, the numbering in accordance with the so-called Kabat system is preferred. The CDR3 of the light chain and, particularly, CDR3 of the heavy chain may constitute the most important determinants in antigen binding within the light and heavy chain variable regions. In some antibody constructs, the heavy chain CDR3 appears to constitute the major area of contact between the antigen and the antibody. In vitro selection schemes in which CDR3 alone is varied can be used to vary the binding properties of an antibody or determine which residues contribute to the binding of an antigen.

"Consisting essentially of" means that the amino acid sequence can vary by about 1, 2, 3, 4, 5, 6, 7, 8, 9, 10, 11, 12, 13, 14, or 15% relative to the recited SEQ ID NO: sequence and still retain biological activity, as described herein.
In some embodiments, the binding molecules of the invention are isolated proteins or substantially pure proteins. An "isolated" protein is unaccompanied by at least some of the material with which it is normally associated in its natural state, for example constituting at least about 5%, or at least about 50% by weight of the total protein in a given sample. It is understood that the isolated protein may constitute from 5 to 99.9% by weight of the total protein content depending on the circumstances. For example, the protein may be made at a significantly higher concentration through the use of an inducible promoter or high expression promoter, such that the protein is made at increased concentration levels. The definition includes the production of an antigen binding protein in a wide variety of organisms and/or host cells that are known in the art.
For amino acid sequences, sequence identity and/or similarity is determined by using standard techniques known in the art, including, but not limited to, the local sequence identity algorithm of Smith and Waterman, 1981, Adv. Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al., 1984, Nucl. Acid Res. 12:387-395, preferably using the default settings, or by inspection. Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.
An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, 1987, J. Mol. Evol. 35:351-360; the method is similar to that described by Higgins and Sharp, 1989, CABIOS 5:151-153. Useful PILEUP parameters including a default gap weight of 3.00, a default gap length weight of 0.10, and weighted end gaps.
Another example of a useful algorithm is the BLAST algorithm, described in: Altschul et al., 1990, J. Mol. Biol. 215:403-410; Altschul et al., 1997, Nucleic Acids Res. 25:3389-3402; and Karin et al., 1993, Proc. Natl. Acad. Sci. U.S.A. 90:5873-5787. A particularly useful BLAST program is the WU-BLAST-2 program which was obtained from Altschul et al., 1996, Methods in Enzymology 266:460-480. WU-BLAST-2 uses several search parameters, most of which are set to the default values. The adjustable parameters are set with the following values: overlap span=1, overlap fraction=0.125, word threshold (T)=II. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., 1993, Nucl. Acids Res. 25:3389-3402. Gapped BLAST uses BLOSUM-62 substitution scores; threshold T parameter set to 9; the two-hit method to trigger ungapped extensions, charges gap lengths of k a cost of 10+k; Xu set to 16, and Xg set to 40 for database search stage and to 67 for the output stage of the algorithms. Gapped alignments are triggered by a score corresponding to about 22 bits.
Generally, the amino acid homology, similarity, or identity between individual variant CDRs are at least 80% to the sequences depicted herein, and more typically with preferably increasing homologies or identities of at least 85%, 90%, 91%, 92%, 93%, 94%, 95%, 96%, 97%, 98%, 99%, and almost 100%. In a similar manner, "percent (%) nucleic acid sequence identity" with respect to the nucleic acid sequence of the binding proteins identified herein is defined as the percentage of nucleotide residues in a candidate sequence that are identical with the nucleotide residues in the coding sequence of the antigen binding protein. A specific method utilizes the BLASTN module of WU-BLAST-2 set to the default parameters, with overlap span and overlap fraction set to 1 and 0.125, respectively.
Generally, the nucleic acid sequence homology, similarity, or identity between the nucleotide sequences encoding individual variant CDRs and the nucleotide sequences depicted herein are at least 80%, and more typically with preferably increasing homologies or identities of at least 80%, 81%, 82%, 83%, 84%, 85%, 86%, 87%, 88%, 89%, 90%, 91%, 92%, 93%, 94%, 95%, 96%, 97%, 98%, or 99%, and almost 100%.
Thus, a "variant CDR" is one with the specified homology, similarity, or identity to the parent CDR of the invention, and shares biological function, including, but not limited to, at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent CDR.
While the site or region for introducing an amino acid sequence variation is predetermined, the mutation *per se* need not be predetermined. For example, in order to optimize the performance of a mutation at a given site, random mutagenesis may be conducted at the target codon or region and the expressed antigen binding protein CDR variants screened for the optimal combination of desired activity. Techniques for making substitution mutations at predetermined sites in DNA having a known sequence are well known, for example, M13 primer mutagenesis and PCR mutagenesis. Screening of the mutants is done using assays of antigen binding protein activities, such as binding to an elected a cell surface molecule on a target cell.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gin or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gin, Gly, His, Met, Phe, Ser, Thr, Trp, and Tyr).

The term "hypervariable region" (also known as "complementarity determining regions" or CDRs) when used herein refers to the amino acid residues of an antibody which are (usually three or four short regions of extreme sequence variability) within the V-region domain of an immunoglobulin which form the antigen-binding site and are the main determinants of antigen specificity. There are at least two methods for identifying the CDR residues: (1) An approach based on cross-species sequence variability (i. e., Kabat *et al.,* loc. cit.); and (2) An approach based on crystallographic studies of antigen-antibody complexes (Chothia, C. etal., J. Mol. Biol. 196: 901-917 (1987)). However, to the extent that two residue identification techniques define regions of overlapping, but not identical regions, they can be combined to define a hybrid CDR. However, in general, the CDR residues are preferably identified in accordance with the so-called Kabat (numbering) system.

The term "framework region" refers to the art-recognized portions of an antibody variable region that exist between the more divergent (i.e., hypervariable) CDRs. Such framework regions are typically referred to as frameworks 1 through 4 (FR1, FR2, FR3, and FR4) and provide a scaffold for the presentation of the six CDRs (three from the heavy chain and three from the light chain) in three dimensional space, to form an antigen-binding surface.

Typically, CDRs form a loop structure that can be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800, each of which is incorporated by reference in its entirety). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues. The term "canonical structure" may also include considerations as to the linear sequence of the antibody, for example, as catalogued by Kabat (Kabat *et al.,* loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al and/or revealed by other techniques, for example, crystallography and two or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia *et al.,* loc. cit. and their implications for construing canonical aspects of antibody structure, are described in the literature.

CDR3 is typically the greatest source of molecular diversity within the antibody-binding site. H3, for example, can be as short as two amino acid residues or greater than 26 amino acids. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. One of skill in the art will recognize that each subunit structure, e.g., a CH, VH, CL, VL, CDR, FR structure, comprises active fragments, e.g., the portion of the VH, VL, or CDR subunit the binds to the antigen, i.e., the antigen-binding fragment, or, e.g., the portion of the CH subunit that binds to and/or activates, e.g., an Fc receptor and/or complement. The CDRs typically refer to the Kabat CDRs, as described in Sequences of Proteins of immunological Interest, US Department of Health and Human Services (1991), eds. Kabat *et al.* Another standard for characterizing the antigen binding site is to refer to the hypervariable loops as described by Chothia. See, e.g., Chothia, et al. (1987; J. Mol. Biol. 227:799-817); and Tomlinson et al. (1995) EMBO J. 14: 4628-4638. Still another standard is the AbM definition used by Oxford Molecular's AbM antibody modeling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Embodiments described with respect to Kabat CDRs can alternatively be implemented using similar described relationships with respect to Chothia hypervariable loops or to the AbM-defined loops.

The sequence of antibody genes after assembly and somatic mutation is highly varied, and these varied genes are estimated to encode 10¹⁰ different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., in vitro stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

The term "binding molecule" or "antibody construct" in the sense of the present disclosure indicates any molecule capable of (specifically) binding to, interacting with or recognizing the target molecules a cell surface molecule on a target cell and CD3. Such molecules or constructs may include proteinaceous parts and non-proteinaceous parts (e.g. chemical linkers or chemical cross-linking agents such as glutaraldehyde).
In the event that a linker is used, this linker is preferably of a length and sequence sufficient to ensure that each of the first, second and third domains can, independently from one another, retain their differential binding specificities. Most preferably and as documented in the appended examples, the binding molecule of the invention is a "bispecific single chain binding molecule", more preferably a bispecific single chain Fv (scFv). Bispecific single chain molecules are known in the art and are described in WO 99/54440, Mack, J. Immunol. (1997), 158, 3965-3970, Mack, PNAS, (1995), 92, 7021-7025, Kufer, Cancer Immunol. Immunother., (1997), 45, 193-197, Loffler, Blood, (2000), 95, 6, 2098-2103, Brühl, Immunol., (2001), 166, 2420-2426, Kipriyanov, J. Mol. Biol., (1999), 293, 41-56.

The said variable domains comprised in the herein described binding molecules may be connected by additional linker sequences. The term "peptide linker" defines in accordance with the present invention an amino acid sequence by which the amino acid sequences of the first domain, the second domain and the third domain of the binding molecule of the invention are linked with each other. An essential technical feature of such peptide linker is that said peptide linker does not comprise any polymerization activity. Among the suitable peptide linkers are those described in U.S. Patents 4,751,180 and 4,935,233 or WO 88/09344. A preferred embodiment of a peptide linker is characterized by the amino acid sequence Gly-Gly-Gly-Gly-Ser, i.e. Gly₄Ser, or polymers thereof, i.e. (Gly₄Ser)x, where x is an integer 1 or greater. The characteristics of said peptide linker, which comprise the absence of the promotion of secondary structures are known in the art and described e.g. in Dall'Acqua et al. (Biochem. (1998) 37, 9266-9273), Cheadle et al. (Mol Immunol (1992) 29, 21-30) and Raag and Whitlow (FASEB (1995) 9(1), 73-80). Peptide linkers which also do not promote any secondary structures are preferred. The linkage of said domains to each other can be provided by, e.g. genetic engineering, as described in the examples. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).
For peptide linkers, which connect the binding domains in the binding molecule of the invention peptide linkers are preferred which comprise only a few number of amino acid residues, e.g. 12 amino acid residues or less. Thus, peptide linker of 12, 11, 10, 9, 8, 7, 6 or 5 amino acid residues are preferred. An envisaged peptide linker with less than 5 amino acids comprises 4, 3, 2 or one amino acid(s) wherein Gly-rich linkers are preferred. A particularly preferred "single" amino acid in context of said "peptide linker" is Gly. Accordingly, said peptide linker may consist of the single amino acid Gly.

The term "multispecific" as used herein refers to a binding molecule of the invention which comprises at least is capable of binding to at least one further antigen or target.

It is also envisaged that the binding molecule of the invention has, apart from addition to the specificity for serum albumin and in addition to its function to bind to a cell surface molecule on a target cell and CD3, a further function. In this format, the antibody construct is a multifunctional antibody construct by targeting cells through binding to a cell surface molecule on a target cell, mediating cytotoxic T cell activity through CD3 binding and providing a further function such as a fully functional Fc constant domain mediating antibody-dependent cellular cytotoxicity through recruitment of effector cells like NK cells, a label (fluorescent etc.), a therapeutic agent such as, e.g. a toxin or radionuclide, and/or means to enhance serum half-life, etc.

The term "binding domain" characterizes in connection with the present invention a domain which is capable of specifically binding to / interacting with a given target epitope or a given target site on the target cell surface molecule on a target cell and CD3.
Binding domains can be derived from a binding domain donor such as for example an antibody. It is envisaged that a binding domain of the present invention comprises at least said part of any of the aforementioned binding domains that is required for binding to/interacting with a given target epitope or a given target site on the cell surface molecule on a target cell and CD3.

It is envisaged that the binding domain of the aforementioned binding domain donors is characterized by that part of these donors that is responsible for binding the respective target, i.e. when that part is removed from the binding domain donor, said donor loses its binding capability. "Loses" means a reduction of at least 50% of the binding capability when compared with the binding donor. Methods to map these binding sites are well known in the art - it is therefore within the standard knowledge of the skilled person to locate/map the binding site of a binding domain donor and, thereby, to "derive" said binding domain from the respective binding domain donors.

The term "epitope" refers to a site on an antigen to which a binding domain, such as an antibody or immunoglobulin or derivative or fragment of an antibody or of an immunoglobulin, specifically binds. An "epitope" is antigenic and thus the term epitope is sometimes also referred to herein as "antigenic structure" or "antigenic determinant". Thus, the binding domain is an "antigen-interaction-site". Said binding/interaction is also understood to define a "specific recognition". In one example, said binding domain which (specifically) binds to / interacts with a given target epitope or a given target site on a cell surface molecule on a target cell and CD3 is an antibody or immunoglobulin, and said binding domain is a VH and/or VL region of an antibody or of an immunoglobulin.
"Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically a conformational epitope comprises an increased number of amino acids relative to a linear epitope. With regard to recognition of conformational epitopes, the binding domain recognizes a three-dimensional structure of the antigen, preferably a peptide or protein or fragment thereof (in the context of the present invention, the antigen for one of the binding domains is comprised within a cell surface molecule on a target cell). For example, when a protein molecule folds to form a three-dimensional structure, certain amino acids and/or the polypeptide backbone forming the conformational epitope become juxtaposed enabling the antibody to recognize the epitope. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy. Moreover, the provided examples describe a further method to characterize a given binding domain by way of binning, which includes a test whether the given binding domain binds to one or more epitope cluster(s) of a given protein, in particular a cell surface molecule on a target cell.
As used herein, the term "epitope cluster" denotes the entirety of epitopes lying in a defined contiguous stretch of an antigen. An epitope cluster can comprise one, two or more epitopes. The concept of epitope cluster is also used in the characterization of the features of the binding molecules of the invention.

The terms "(capable of) binding to", "specifically recognizing", "directed to" and "reacting with" mean in accordance with this invention that a binding domain is capable of specifically interacting with one or more, preferably at least two, more preferably at least three and most preferably at least four amino acids of an epitope.

As used herein, the terms "specifically interacting", "specifically binding" or "specifically bind(s)" mean that a binding domain exhibits appreciable affinity for a particular protein or antigen and, generally, does not exhibit significant reactivity with proteins or antigens other than the cell surface molecule on a target cell or CD3. "Appreciable affinity" includes binding with an affinity of about 10⁻⁶M (KD) or stronger. Preferably, binding is considered specific when binding affinity is about 10⁻¹² to 10⁻⁸ M, 10⁻¹² to 10⁻⁹ M, 10⁻¹² to 10⁻¹⁰ M, 10⁻¹¹ to 10⁻⁸ M, preferably of about 10⁻¹¹ to 10⁻⁹ M. Whether a binding domain specifically reacts with or binds to a target can be tested readily by, *inter alia,* comparing the reaction of said binding domain with a target protein or antigen with the reaction of said binding domain with proteins or antigens other than the cell surface molecule on a target cell or CD3. Preferably, a binding domain of the invention does not essentially bind or is not capable of binding to proteins or antigens other than the cell surface molecule on a target cell or CD3 (i.e. the second binding domain is not capable of binding to proteins other than the cell surface molecule on a target cell and the third binding domain is not capable of binding to proteins other than CD3).
The term "does not essentially bind", or "is not capable of binding" means that a binding domain of the present invention does not bind another protein or antigen other than the elected cell surface molecule on a target cell or CD3, i.e., does not show reactivity of more than 30%, preferably not more than 20%, more preferably not more than 10%, particularly preferably not more than 9%, 8%, 7%, 6% or 5% with proteins or antigens other than the cell surface molecule on a target cell or CD3, whereby binding to the cell surface molecule on a target cell or CD3, respectively, is set to be 100%.
Specific binding is believed to be effected by specific motifs in the amino acid sequence of the binding domain and the antigen. Thus, binding is achieved as a result of their primary, secondary and/or tertiary structure as well as the result of secondary modifications of said structures. The specific interaction of the antigen-interaction-site with its specific antigen may result in a simple binding of said site to the antigen. Moreover, the specific interaction of the antigen-interaction-site with its specific antigen may alternatively or additionally result in the initiation of a signal, e.g. due to the induction of a change of the conformation of the antigen, an oligomerization of the antigen, etc.

Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. A "polypeptide" when referred to herein may also be chemically modified such as pegylated. Such modifications are well known in the art.

"Isolated" when used to describe the binding molecule disclosed herein, means a binding molecule that has been identified, separated and/or recovered from a component of its production environment. Preferably, the isolated binding molecule is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the binding molecule will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

Amino acid sequence modifications of the binding molecules described herein are contemplated. For example, it may be desirable to improve the binding affinity and/or other biological properties of the antibody. Amino acid sequence variants of the binding molecules are prepared by introducing appropriate nucleotide changes into the binding molecules nucleic acid, or by peptide synthesis.

Such modifications include, for example, deletions from, and/or insertions into, and/or substitutions of, residues within the amino acid sequences of the binding molecules. Any combination of deletion, insertion, and substitution is made to arrive at the final construct, provided that the final construct possesses the desired characteristics. The amino acid changes also may alter post-translational processes of the binding molecules, such as changing the number or position of glycosylation sites. Preferably, 1, 2, 3, 4, 5, 6, 7, 8, 9, or 10 amino acids may be substituted in a CDR, while 1, 2, 3, 4, 5, 6, 7, 8, 9, 10, 11, 12, 13, 14, 15, 16, 17, 18, 19, 20, or 25 amino acids may be substituted in the framework regions (FRs). The substitutions are preferably conservative substitutions as described herein. Additionally or alternatively, 1, 2, 3, 4, 5, or 6 amino acids may be inserted or deleted in each of the CDRs (of course, dependent on their length), while 1, 2, 3, 4, 5, 6, 7, 8, 9, 10, 11, 12, 13, 14, 15, 16, 17, 18, 19, 20, or 25 amino acids may be inserted or deleted in each of the FRs.

A useful method for identification of certain residues or regions of the binding molecules that are preferred locations for mutagenesis is called "alanine scanning mutagenesis" as described by Cunningham and Wells in Science, 244: 1081-1085 (1989). Here, a residue or group of target residues within the binding molecule is/are identified (e.g. charged residues such as arg, asp, his, lys, and glu) and replaced by a neutral or negatively charged amino acid (most preferably alanine or polyalanine) to affect the interaction of the amino acids with the epitope.

Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* needs not to be predetermined. For example, to analyze the performance of a mutation at a given site, ala scanning or random mutagenesis is conducted at a target codon or region and the expressed binding molecule variants are screened for the desired activity.

Preferably, amino acid sequence insertions include amino- and/or carboxyl-terminal fusions ranging in length from 1, 2, 3, 4, 5, 6, 7, 8, 9 or 10 residues to polypeptides containing a hundred or more residues, as well as intrasequence insertions of single or multiple amino acid residues. An insertional variant of the binding molecule includes the fusion to the N-or C-terminus of the antibody to an enzyme or a fusion to a polypeptide which increases the serum half-life of the antibody.

Another type of variant is an amino acid substitution variant. These variants have preferably at least 1, 2, 3, 4, 5, 6, 7, 8, 9 or 10 amino acid residues in the binding molecule replaced by a different residue. The sites of greatest interest for substitutional mutagenesis include the CDRs of the heavy and/or light chain, in particular the hypervariable regions, but FR alterations in the heavy and/or light chain are also contemplated.

For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

Generally, if amino acids are substituted in one or more or all of the CDRs of the heavy and/or light chain, it is preferred that the then-obtained "substituted" sequence is at least 60%, more preferably 65%, even more preferably 70%, particularly preferably 75%, more particularly preferably 80% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the binding molecule may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions" listed in Table 1, below) is envisaged as long as the binding molecule retains its capability to bind to the cell surface molecule on a target cell via the second binding domain and to CD3 epsilon via the third binding domain and/or its CDRs have an identity to the then substituted sequence (at least 60%, more preferably 65%, even more preferably 70%, particularly preferably 75%, more particularly preferably 80% identical to the "original" CDR sequence).

Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino Acid Substitutions**

| Original | Exemplary Substitutions | Preferred Substitutions |
|---|---|---|
| Ala (A) | val, leu, ile | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | arg |
| Ile (I) | leu, val, met, ala, phe | leu |
| Leu (L) | norleucine, ile, val, met, ala | ile |
| Lys (K) | arg, gln, asn | arg |
| Met (M) | leu, phe, ile | leu |
| Phe (F) | leu, val, ile, ala, tyr | tyr |
| Pro (P) | ala | ala |
| Ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Substantial modifications in the biological properties of the binding molecule of the present invention are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr; (3) acidic: asp, glu; (4) basic: asn, gin, his, lys, arg; (5) residues that influence chain orientation: gly, pro; and (6) aromatic : trp, tyr, phe.

Non-conservative substitutions will entail exchanging a member of one of these classes for another class. Any cysteine residue not involved in maintaining the proper conformation of the binding molecule may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e. g. 6-7 sites) are mutated to generate all possible amino acid substitutions at each site. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the binding domain and, e.g., a human cell surface molecule on a target cell. Such contact residues and neighbouring residues are candidates for substitution according to the techniques elaborated herein. Once such variants are generated, the panel of variants is subjected to screening as described herein and antibodies with superior properties in one or more relevant assays may be selected for further development.

Other modifications of the binding molecule are contemplated herein. For example, the binding molecule may be linked to one of a variety of non-proteinaceous polymers, e.g., polyethylene glycol, polypropylene glycol, polyoxyalkylenes, or copolymers of polyethylene glycol and polypropylene glycol. The binding molecule may also be entrapped in microcapsules prepared, for example, by coacervation techniques or by interfacial polymerization (for example, hydroxymethylcellulose or gelatine-microcapsules and poly (methylmethacylate) microcapsules, respectively), in colloidal drug delivery systems (for example, liposomes, albumin microspheres, microemulsions, nanoparticles and nanocapsules), or in macroemulsions. Such techniques are disclosed in Remington's Pharmaceutical Sciences, 16th edition, Oslo, A., Ed., (1980).

The binding molecules disclosed herein may also be formulated as immuno-liposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA, 82: 3688 (1985); Hwang et al. , Proc. Natl Acad. Sci. USA, 77: 4030 (1980); US Pat. Nos. 4,485,045 and 4,544,545; and WO 97/38731 published October 23, 1997. Liposomes with enhanced circulation time are disclosed in US Patent No. 5,013, 556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81 (19) 1484 (1989).

When using recombinant techniques, the binding molecule can be produced intracellularly, in the periplasmic space, or directly secreted into the medium. If the binding molecule is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E. *coli.*

The binding molecule composition prepared from the cells can be purified using, for example, hydroxylapatite chromatography, gel electrophoresis, dialysis, and affinity chromatography, with affinity chromatography being the preferred purification technique.

The term "nucleic acid" is well known to the skilled person and encompasses DNA (such as cDNA) and RNA (such as mRNA). The nucleic acid can be double stranded and single stranded, linear and circular. Said nucleic acid molecule is preferably comprised in a vector which is preferably comprised in a host cell. Said host cell is, e.g. after transformation or transfection with the nucleic acid sequence of the invention, capable of expressing the binding molecule. For that purpose the nucleic acid molecule is operatively linked with control sequences.

A vector is a nucleic acid molecule used as a vehicle to transfer (foreign) genetic material into a cell. The term "vector" encompasses - but is not restricted to - plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences such as a promoter sequence that drives expression of the transgene. Insertion of a vector into the target cell is usually called "transformation" for bacteria, "transfection" for eukaryotic cells, although insertion of a viral vector is also called "transduction".

As used herein, the term "host cell" is intended to refer to a cell into which a nucleic acid encoding the binding molecule of the invention is introduced by way of transformation, transfection and the like. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

As used herein, the term "expression" includes any step involved in the production of a binding molecule of the invention including, but not limited to, transcription, post-transcriptional modification, translation, post-translational modification, and secretion.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The terms "host cell," "target cell" or "recipient cell" are intended to include any individual cell or cell culture that can be or has/have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacteria, yeast cells, animal cells, and mammalian cells, e.g., murine, rat, macaque or human.

Suitable host cells include prokaryotes and eukaryotic host cells including yeasts, fungi, insect cells and mammalian cells.

The binding molecule of the invention can be produced in bacteria. After expression, the binding molecule of the invention, preferably the binding molecule is isolated from the *E. coli* cell paste in a soluble fraction and can be purified through, e.g., affinity chromatography and/or size exclusion. Final purification can be carried out similar to the process for purifying antibody expressed e. g, in CHO cells.

In addition to prokaryotes, eukaryotic microbes such as filamentous fungi or yeast are suitable cloning or expression hosts for the binding molecule of the invention. *Saccharomyces cerevisiae,* or common baker's yeast, is the most commonly used among lower eukaryotic host microorganisms. However, a number of other genera, species, and strains are commonly available and useful herein, such as *Schizosaccharomyces* pombe, Kluyveromyces hosts such as, e.g., *K. lactis, K. fragilis* (ATCC 12424), *K. bulgaricus* (ATCC 16045), *K. wickeramii* (ATCC 24178), *K. waltii* (ATCC 56500), *K. drosophilarum* (ATCC 36906), *K. thermotolerans,* and *K. marxianus;* yarrowia (EP 402 226); *Pichia pastoris* (EP 183 070); Candida; *Trichoderma reesia* (EP 244 234); *Neurospora crassa;* Schwanniomyces such as *Schwanniomyces occidentalis;* and filamentous fungi such as, e.g., Neurospora, Penicillium, Tolypocladium, and Aspergillus hosts such as A. *nidulans* and A. *niger.*

Suitable host cells for the expression of glycosylated binding molecule of the invention, preferably antibody derived binding molecules are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruit fly), and *Bombyx mori* have been identified. A variety of viral strains for transfection are publicly available, e. g. , the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, Arabidopsis and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al. , J. Gen Virol. 36 : 59 (1977)); baby hamster kidney cells (BHK, ATCC CCL 10); Chinese hamster ovary cells/-DHFR (CHO, Urlaub et al. , Proc. Natl. Acad. Sci. USA 77: 4216 (1980)); mouse sertoli cells (TM4, Mather, Biol. Reprod. 23: 243-251 (1980)); monkey kidney cells (CVI ATCC CCL 70); African green monkey kidney cells (VERO-76, ATCC CRL1587) ; human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. 383 : 44-68 (1982)) ; MRC 5 cells; FS4 cells; and a human hepatoma line (Hep G2).

When using recombinant techniques, the binding molecule of the invention can be produced intracellularly, in the periplasmic space, or directly secreted into the medium. If the binding molecule is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The binding molecule of the invention prepared from the host cells can be purified using, for example, hydroxylapatite chromatography, gel electrophoresis, dialysis, and affinity chromatography, with affinity chromatography being the preferred purification technique.

The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose. Where the binding molecule of the invention comprises a CH3 domain, the Bakerbond ABXMresin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromato-focusing, SDS-PAGE, and ammonium sulfate precipitation are also available depending on the antibody to be recovered.

The term "culturing" refers to the in vitro maintenance, differentiation, growth, proliferation and/or propagation of cells under suitable conditions in a medium.

As used herein, the term "pharmaceutical composition" relates to a composition for administration to a patient, preferably a human patient. The particular preferred pharmaceutical composition of this invention comprises the binding molecule of the invention. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient. The pharmaceutical composition comprising the binding molecule of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of these binding molecules of the invention may be intravenous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The inventive compositions may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. In general, as used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counter-ions, such as sodium, polyhydric sugar alcohols; amino acids, such as alanine, glycine, asparagine, 2-phenylalanine, and threonine; sugars or sugar alcohols, such as trehalose, sucrose, octasulfate, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate.

The compositions of the present invention can be administered to the subject at a suitable dose which can be determined e.g. by dose escalating studies by administration of increasing doses of the polypeptide of the invention exhibiting cross-species specificity described herein to non-chimpanzee primates, for instance macaques. As set forth above, the binding molecule of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the polypeptide of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the polypeptide of the invention defined herein, further biologically active agents, depending on the intended use of the composition. Such agents might be drugs acting on the gastro-intestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art. It is also envisaged that the binding molecule of the present invention is applied in a co-therapy, i.e., in combination with another anti-cancer medicament.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1-12). "Efficacy" or *"in vivo* efficacy" as used herein refers to the response to therapy by the pharmaceutical composition of the invention, using e.g. standardized NCI response criteria. The success or *in vivo* efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that affect the ability of a particular drug to treat a given condition, can be established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

"Half-life" means the time where 50% of an administered drug are eliminated through biological processes, e.g. metabolism, excretion, etc.

By "hepatic first-pass metabolism" is meant the propensity of a drug to be metabolized upon first contact with the liver, i.e. during its first pass through the liver.

"Volume of distribution" means the degree of retention of a drug throughout the various compartments of the body, like e.g. intracellular and extracellular spaces, tissues and organs, etc. and the distribution of the drug within these compartments.

"Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma.

"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetic parameters of bispecific single chain antibodies exhibiting cross-species specificity, which may be determined in preclinical animal testing in non-chimpanzee primates as outlined above, are also set forth e.g. in the publication by Schlereth et al. (Cancer Immunol. Immunother. 20 (2005), 1-12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", *"in vivo* safety" or "tolerability" as used herein defines the administration of a drug without inducing severe adverse events directly after administration (local tolerance) and during a longer period of application of the drug. "Safety", *"in vivo* safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviations to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance hematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the infection and the general state of the subject's own immune system. The term "patient" includes human and other mammalian subjects that receive either prophylactic or therapeutic treatment.

The term "effective and non-toxic dose" as used herein refers to a tolerable dose of an inventive binding molecule which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

The appropriate dosage, or therapeutically effective amount, of the binding molecule of the invention will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies such as anti-cancer therapies as needed.

The pharmaceutical compositions of this invention are particularly useful for parenteral administration, i.e., subcutaneously, intramuscularly, intravenously, intra-articular and/or intra-synovial. Parenteral administration can be by bolus injection or continuous infusion.

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

The mode of action of the binding molecules that binds both to a cell surface molecule on a target cell such as a tumor antigen and to the T cell CD3 receptor complex is commonly known. Bringing a T cell in close vicinity to a target cell, i.e., engaging said T cell results under the circumstances in killing of the target cell by the T cell. This process can be exploited in fighting against proliferative disease, inflammatory disease, infectious disease and autoimmune disease. Thus, fusing anything such as additional amino acid sequences to the CD3 binding domain, i.e., the "effector domain" of a binding molecule or to the target binding domain influences the properties of the binding molecule such that it would no longer exert its function in properly engaging a T cell and/or binding to its target. Indeed, T-cells are equipped with granules containing a deadly combination of pore-forming proteins, called perforins, and cell death-inducing proteases, called granzymes. These proteins are delivered into target cells via a cytolytic synapse that can only form if T-cells are in closest vicinity with a target cell that is aimed to be killed. Normally, closest vicinity between a T cell and a target cell is achieved by the T cell binding to an MHC class I/peptide complex using its matching T-cell receptor. Yet, it is the function of the binding molecules of the present invention to bring a T cell into such close vicinity to a target cell in the absence of T cell receptor/MHC interaction. Hence, one can imagine that fusing anything such as additional amino acid sequences to either or all of the first, second and/or third binding domain of the binding molecules of the present invention could negatively influence the function thereof, i.e., bringing together a target cell and a T cell in order to kill the target cell.

That being so and bearing in mind that it is highly desirable to increase the serum half-life of the binding molecules in order to stabilize it or prevent it from fast renal clearance and the like, the skilled person seems to be in a dilemma. Indeed, while an increase of the half-life could be achieved by having a binding molecule binding to, e.g., serum albumin which requires equipping said binding molecule with a domain which is capable of binding to serum albumin, the addition of such a domain could probably adversely affect the properties of said binding molecule, e.g., it might lose its function or become at least less effective.

Notwithstanding this potential dilemma and bearing in mind that a binding molecule of the present invention could at least be weakened or even inactivated by the addition of a further binding which is capable of binding to serum albumin, the present inventors generated binding molecules that have, in addition a binding domain which is capable of binding to a cell surface molecule on a target cell and a binding domain which is capable of binding to the T cell CD3 receptor complex, an additional binding domain which is capable of binding to serum albumin.

WO 01/45746 which provides serum albumin binding domains leaves it completely up to the skilled person to which terminus of a protein, in particular an antibody, a serum albumin binding domain should be fused - it can either be the N-or C-terminus and may depend on the circumstances. Thus, the prior art does not provide guidance.

It has been surprisingly found that the albumin binding domain positioned at the C-terminus of the CD3 receptor specific domain relates to poor yields of monomeric molecules isolated from the supernatant of host cells producing the binding domain. In contrast, when the albumin binding domain was positioned at the N-terminus of the molecule, a significant increase in expression productivity and yield was observed in comparison with the above mentioned binding molecules having the albumin binding domain positioned at the C-terminus of the CD3 receptor.

This increase of the yield of monomeric molecules is indeed a surprise, since one could more likely have expected that adding the identical short albumin binding domain to either the N-terminus or the C-terminus of a given binging molecule with two different specificities for cell surface antigens should have similar effects.

The observed higher yield of monomeric molecules is especially relevant in view of a commercial production of binding molecules of the invention. This optimization in the product quality allows for higher compound concentration in working solutions as well in final pharmaceutical formulations of the binding molecules. It further allows for smaller fermenter volumes resulting in the same production yield and for lesser cost for purification columns and other equipment for manufacturing the product and, consequently for preferred cost of goods calculation. Moreover, the higher production concentration/concentration of product in the production cell supernatant allows for more stringent purification/isolation procedures with higher acceptable loss of product during the production, which leads to a clear separation of the product from undesired components and, thus, to a preferred higher product purity.

All the more, prior art binding molecules, such as diabodies with two binding domains, one for CD3 and a second for a target molecule such as CEA, and equipped with a serum albumin binding domain are constructed in a way that the serum albumin binding domain is fused to the C-terminus of the domain binding to the target cell; see Stork et al., Prot Eng Des Sel 20(11), 569-576 (2007) and Mueller et al., J Biol Chem 282(17), 12650-12660 (2007). Thus, also from the prior one would have concluded that a serum albumin binding domain should be added to the C-terminus of the domain binding to a cell surface molecule of target cell. A similar approach was done in the construction of DART antibodies such as an ABD-DART (see WO 2010/080538, e.g. Figure 45).

That being so, the present inventors, despite the teaching of the prior art and the expectations grasped from the teaching of the prior art, added a serum albumin binding domain to the N-terminus of the binding domain that engages a target cell via binding to a cell surface molecule with a T cell via binding to the T cell receptor complex and were successful in the generation of a binding molecule that has an increased serum half-life in a desired yield, while it is still capable of binding to a cell surface molecule on a target cell and binding to the T cell CD3 receptor complex. Thereby it is still feasible engaging the T cell in a way that it exerts its killing functions on the target cell. Thus, a binding molecule of the present invention in which the binding domains are in the order as described herein (see, e.g. claim 1) is capable of mediating cytotoxicity on a target cell that is effected by T cell engaged by said binding molecule.

Due to the albumin binding domain of the binding molecule of the present invention, a binding molecule of the present invention has preferably an increased half-life and/or longer persistence times in the body, thereby also providing a longer functional activity of the binding molecule while it is still producible in an amount desirable for commercial production scale.

In addition, the present inventors have observed that a binding molecule of the present invention is also capable of mediating cytotoxicity in vitro in the presence of 10% (v/v) serum albumin, in particular human serum albumin. This is an important feature, since in human blood serum albumin is present at about 10-20% (v/v). In fact, a binding molecule of the present invention is not-naturally occurring in a mammal, in particular in a human and, thus, it could well have been that the presence of serum albumin could somehow disturb or interfere with the action of a binding molecule of the present invention.

By way of example, the in vitro cytotoxicity assay in the presence of serum albumin, in particular human serum albumin can be used to test a binding molecule of the present invention for its capability of mediating cytotoxicity.

Another surprising property of a binding molecule of the present invention having the order (set-up/arrangement) of the domains as described herein, e.g. in claim 1, can mainly be produced in high yields as monomer. In particular, the present inventors found that more than 80, 85, 90 or even 95% of the binding molecule obtainable from host cells expressing said binding molecule are in the form of a monomer. This is an important feature, since dimers or even multimers are not desirable since they are assumed to have lost most of their binding capabilities to a target cell (via a cell surface molecule) and/or a T cell (via the T cell receptor complex).

In view of the above, the present invention provides an isolated single chain binding molecule comprising at least three binding domains, wherein
(a) the first binding domain is capable of binding to serum albumin and is positioned at the N-terminus of the second binding domain;
(b) said second binding domain is capable of binding to a cell surface molecule on a target cell; and
(c) the third binding domain is capable of binding to the T cell CD3 receptor complex.

The term "cell surface molecule on a target cell" or "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located in the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface molecules which are located on the plasma membrane are proteins which have been modified at a cysteine residue to bear a palmitoyl group, proteins modified at a C-terminal cysteine residue to bear a farnesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies. As apparent from the appended examples, non-limiting examples for cell surface molecules on a target cell are the CD33 molecule and the CEA molecule. Binding domains for CD33 suitable for the herein described format of a binding molecule are described in detail e.g. in WO 2008/119567. Corresponding binding domains for CEA suitable for the herein described format of a binding molecule are described in detail e.g. in WO 2007/071426.

The T cell CD3 receptor complex is a protein complex and is composed of four distinct chains. In mammals, the complex contains a CD3γ chain, a CD3δ chain, and two CD3ε (epsilon) chains. These chains associate with a molecule known as the T cell receptor (TCR) and the ζ chain to generate an activation signal in T lymphocytes.

The redirected lysis of target cells via the recruitment of T cells by a multispecific, at least bispecific, binding molecule involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

The affinity of the second binding domain for a human cell surface molecule on a target cell is preferably ≤100 nM and more preferably ≤50 nM. In a preferred embodiment of the invention the affinity is ≤15 nM, more preferably ≤10 nM, even more preferably ≤5 nM, even more preferably ≤1 nM, even more preferably ≤0.5 nM, even more preferably ≤0.1 nM, and most preferably ≤0.05 nM. The affinity of the second binding domain for a macaque cell surface molecule on a target cell is preferably ≤100 nM and more preferably ≤50 nM. In a preferred embodiment of the invention the affinity is ≤15 nM, more preferably ≤10 nM, even more preferably ≤5 nM, even more preferably ≤1 nM, even more preferably ≤0.5 nM, even more preferably :50.1 nM, and most preferably ≤0.05 nM or even ≤0.01 nM. The affinity can be measured for example in a Biacore assay or in a Scatchard assay, e.g. as described in the Examples. The affinity gap for binding to macaque cell surface molecule on a target cell versus human cell surface molecule on a target cell is preferably [1:10-1:5] or [5:1-10:1], more preferably [1:5-5:1], and most preferably [1:2-3:1] or even [1:1-3:1]. Other methods of determining the affinity are well-known to the skilled person.

Human antibodies, respectively human binding molecules, avoid some of the problems associated with antibodies/binding molecules that possess murine or rat variable and/or constant regions. The presence of such murine or rat derived proteins can lead to the rapid clearance of the antibodies/binding molecules or can lead to the generation of an immune response against the antibody/binding molecule by a patient. In order to avoid the utilization of murine or rat derived antibodies/binding molecules, human or fully human antibodies can be generated through the introduction of human antibody function into a rodent so that the rodent produces fully human antibodies.

The ability to clone and reconstruct megabase-sized human loci in YACs and to introduce them into the mouse germline provides a powerful approach to elucidating the functional components of very large or crudely mapped loci as well as generating useful models of human disease. Furthermore, the utilization of such technology for substitution of mouse loci with their human equivalents could provide unique insights into the expression and regulation of human gene products during development, their communication with other systems, and their involvement in disease induction and progression.
An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B-cell development. Furthermore, such a strategy could provide an ideal source for production of fully human monoclonal antibodies (mAbs)--an important milestone towards fulfilling the promise of antibody therapy in human disease. Fully human antibodies/binding molecules are expected to minimize the immunogenic and allergic responses intrinsic to mouse or mouse-derivatized mAbs and thus to increase the efficacy and safety of the administered antibodies/binding molecules. The use of fully human antibodies/binding molecules can be expected to provide a substantial advantage in the treatment of chronic and recurring human diseases, such as inflammation, autoimmunity, and cancer, which require repeated compound administrations. One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against any antigen of interest, including human antigens. Using the hybridoma technology, antigen-specific human mAbs with the desired specificity could be readily produced and selected. This general strategy was demonstrated in connection with our generation of the first XenoMouse mouse strains, as published in 1994. (See Green et al. Nature Genetics 7:13-21 (1994)) The XenoMouse strains were engineered with yeast artificial chromosomes (YACs) containing 245 kb and 190 kb-sized germline configuration fragments of the human heavy chain locus and kappa light chain locus, respectively, which contained core variable and constant region sequences. Id. The human Ig containing YACs proved to be compatible with the mouse system for both rearrangement and expression of antibodies and were capable of substituting for the inactivated mouse Ig genes. This was demonstrated by their ability to induce B-cell development, to produce an adult-like human repertoire of fully human antibodies, and to generate antigen-specific human mAbs. These results also suggested that introduction of larger portions of the human Ig loci containing greater numbers of V genes, additional regulatory elements, and human Ig constant regions might recapitulate substantially the full repertoire that is characteristic of the human humoral response to infection and immunization. The work of Green et al. was recently extended to the introduction of greater than approximately 80% of the human antibody repertoire through introduction of megabase sized, germline configuration YAC fragments of the human heavy chain loci and kappa light chain loci, respectively. See Mendez et al. Nature Genetics 15:146-156 (1997) and U.S. patent application Ser. No. 08/759,620, filed Dec. 3, 1996, the disclosures of which are hereby incorporated by reference.
The production of the XenoMouse mice is further discussed and delineated in U.S. patent application Ser. No. 07/466,008, filed Jan. 12, 1990, Ser. No. 07/610,515, filed Nov. 8, 1990, Ser. No. 07/919,297, filed Jul. 24, 1992, Ser. No. 07/922,649, filed Jul. 30, 1992, filed Ser. No. 08/031,801, filed Mar. 15, 1993, Ser. No. 08/112,848, filed Aug. 27, 1993, Ser. No. 08/234,145, filed Apr. 28, 1994, Ser. No. 08/376,279, filed Jan. 20, 1995, Ser. No. 08/430,938, Apr. 27, 1995, Ser. No. 08/464,584, filed Jun. 5, 1995, Ser. No. 08/464,582, filed Jun. 5, 1995, Ser. No. 08/463,191, filed Jun. 5, 1995, Ser. No. 08/462,837, filed Jun. 5, 1995, Ser. No. 08/486,853, filed Jun. 5, 1995, Ser. No. 08/486,857, filed Jun. 5, 1995, Ser. No. 08/486,859, filed Jun. 5, 1995, Ser. No. 08/462,513, filed Jun. 5, 1995, Ser. No. 08/724,752, filed Oct. 2, 1996, and Ser. No. 08/759,620, filed Dec. 3, 1996 and U.S. Pat. Nos. 6,162,963, 6,150,584, 6,114,598, 6,075,181, and 5,939,598 and Japanese Patent Nos. 3 068 180 B2, 3 068 506 B2, and 3 068 507 B2. See also Mendez et al. Nature Genetics 15:146-156 (1997) and Green and Jakobovits J. Exp. Med. 188:483-495 (1998). See also European Patent No., EP 0 463151 B1, grant published Jun. 12, 1996, International Patent Application No., WO 94/02602, published Feb. 3, 1994, International Patent Application No., WO 96/34096, published Oct. 31, 1996, WO 98/24893, published Jun. 11, 1998, WO 00/76310, published Dec. 21, 2000, WO 03/47336. The disclosures of each of the above-cited patents, applications, and references are hereby incorporated by reference in their entirety.
In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V.sub.H genes, one or more D.sub.H genes, one or more J.sub.H genes, a mu constant region, and a second constant region (preferably a gamma constant region) are formed into a construct for insertion into an animal. This approach is described in U.S. Pat. No. 5,545,807 to Surani et al. and U.S. Pat. Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Pat. Nos. 5,591,669 and 6,023.010 to Krimpenfort and Berns, U.S. Pat. Nos. 5,612,205, 5,721,367, and 5,789,215 to Berns et al., and U.S. Pat. No. 5,643,763 to Choi and Dunn, and GenPharm International U.S. patent application Ser. No. 07/574,748, filed Aug. 29, 1990, Ser. No. 07/575,962, filed Aug. 31, 1990, Ser. No. 07/810,279, filed Dec. 17, 1991, Ser. No. 07/853,408, filed Mar. 18, 1992, Ser. No. 07/904,068, filed Jun. 23, 1992, Ser. No. 07/990,860, filed Dec. 16, 1992, Ser. No. 08/053,131, filed Apr. 26, 1993, Ser. No. 08/096,762, filed Jul. 22, 1993, Ser. No. 08/155,301, filed Nov. 18, 1993, Ser. No. 08/161,739, filed Dec. 3, 1993, Ser. No. 08/165,699, filed Dec. 10, 1993, Ser. No. 08/209,741, filed Mar. 9, 1994, the disclosures of which are hereby incorporated by reference. See also European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Pat. No. 5,981,175, the disclosures of which are hereby incorporated by reference in their entirety. See further Taylor et al., 1992, Chen et al., 1993, Tuaillon et al., 1993, Choi et al., 1993, Lonberg et al., (1994), Taylor et al., (1994), and Tuaillon et al., (1995), Fishwild et al., (1996), the disclosures of which are hereby incorporated by reference in their entirety.
Kirin has also demonstrated the generation of human antibodies from mice in which, through microcell fusion, large pieces of chromosomes, or entire chromosomes, have been introduced. See European Patent Application Nos. 773 288 and 843 961, the disclosures of which are hereby incorporated by reference.Xenerex Biosciences is developing a technology for the potential generation of human antibodies. In this technology, SCID mice are reconstituted with human lymphatic cells, e.g., B and/or T cells. Mice are then immunized with an antigen and can generate an immune response against the antigen. See U.S. Pat. Nos. 5,476,996, 5,698,767, and 5,958,765.
Human anti-mouse antibody (HAMA) responses have led the industry to prepare chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. Thus, it would be desirable to provide fully human antibodies against EGFRvlll in order to vitiate concerns and/or effects of HAMA or HACA response.

Cytotoxicity mediated by anti-cell surface molecule/CD3 bispecific binding molecules can be measured in various ways. Effector cells can be e.g. stimulated enriched (human) CD8 positive T cells or unstimulated (human) peripheral blood mononuclear cells (PBMC). If the target cells are of macaque origin or express or are transfected with macaque cell surface molecule, the effector cells should also be of macaque origin such as a macaque T cell line, e.g. 4119LnPx. The target cells should express (at least the extracellular domain of) cell surface molecule, e.g. the human or macaque cell surface molecule. Target cells can be a cell line (such as CHO) which is stably or transiently transfected with the cell surface molecule, e.g. the human or the macaque cell surface molecule. Alternatively, the target cells can be an elected cell surface molecule positive natural expresser cell line. Thus, if e.g. the elected cell surface molecule is CD33, the target cells must express CD33, either as target cells naturally expressing the CD33 molecule or, as described, after transfection of the correseponding gene in the format of an expression vector. Usually EC₅₀-values are expected to be lower with target cell lines expressing higher levels of the elected cell surface molecule on the cell surface. The effector to target cell (E:T) ratio is usually about 10:1, but can also vary. Cytotoxic activity of anti-cell surface molecule/CD3 bispecific binding molecules can be measured in a ⁵¹-chromium release assay (incubation time of about 18 hours) or in a in a FACS-based cytotoxicity assay (incubation time of about 48 hours). Modifications of the assay incubation time (cytotoxic reaction) are also possible. Other methods of measuring cytotoxicity are well-known to the skilled person and comprise MTT or MTS assays, ATP-based assays including bioluminescent assays, the sulforhodamine B (SRB) assay, WST assay, clonogenic assay and the ECIS technology.

The cytotoxic activity mediated by anti-cell surface molecule/CD3 bispecific binding molecules of the present invention is preferably measured in a cell-based cytotoxicity assay. It is represented by the EC₅₀ value, which corresponds to the half maximal effective concentration (concentration of the binding molecule which induces a cytotoxic response halfway between the baseline and maximum). Preferably, the EC₅₀ value of the anti-cell surface molecule/CD3 bispecific binding molecules is ≤20.000 pg/ml, more preferably ≤5000 pg/ml, even more preferably ≤1000 pg/ml, even more preferably ≤500 pg/ml, even more preferably ≤350 pg/ml, even more preferably ≤320 pg/ml, even more preferably ≤250 pg/ml, even more preferably ≤100 pg/ml, even more preferably ≤50 pg/ml, even more preferably ≤10 pg/ml, and most preferably ≤5 pg/ml.

Any of the above given EC₅₀ values can be combined with any one of the indicated scenarios of a cell-based cytotoxicity assay. For example, when (human) CD8 positive T cells or a macaque T cell line are used as effector cells, the EC₅₀ value of the anti-cell surface molecule/CD3 bispecific binding molecule is preferably ≤1000 pg/ml, more preferably ≤500 pg/ml, even more preferably ≤250 pg/ml, even more preferably ≤100 pg/ml, even more preferably ≤50 pg/ml, even more preferably ≤10 pg/ml, and most preferably ≤5 pg/ml. If in this assay the target cells are (human or macaque) elected cell surface molecule transfected cells such as CHO cells, the EC₅₀ value of the anti-cell surface molecule/CD3 bispecific binding molecule is preferably ≤150 pg/ml, more preferably ≤100 pg/ml, even more preferably ≤50 pg/ml, even more preferably ≤30 pg/ml, even more preferably ≤10 pg/ml, and most preferably ≤5 pg/ml.
If the target cells are elected cell surface molecule positive natural expresser cell line, then the EC₅₀ value is preferably ≤350 pg/ml, more preferably ≤320 pg/ml, even more preferably ≤250 pg/ml, even more preferably ≤200 pg/ml, even more preferably ≤100 pg/ml, even more preferably ≤150 pg/ml, even more preferably ≤100 pg/ml, and most preferably ≤50 pg/ml, or lower.
When (human) PBMCs are used as effector cells, the EC₅₀ value of the anti-cell surface molecule/CD3 bispecific binding molecule is preferably ≤1000 pg/ml, more preferably ≤750 pg/ml, more preferably ≤500 pg/ml, even more preferably ≤350 pg/ml, even more preferably ≤320 pg/ml, even more preferably ≤250 pg/ml, even more preferably ≤100 pg/ml, and most preferably ≤50 pg/ml, or lower.

The difference in cytotoxic activity between the monomeric and the dimeric isoform of individual anti-cell surface molecule/CD3 bispecific binding molecules is referred to as "potency gap". This potency gap can e.g. be calculated as ratio between EC₅₀ values of the molecule's monomeric and dimeric form. Potency gaps of the anti-cell surface molecule/CD3 bispecific binding molecules of the present invention are preferably ≤5, more preferably ≤4, even more preferably ≤3, even more preferably ≤2 and most preferably ≤1.

The binding molecule of the invention is a fusion protein comprising at least two binding domains, with or without peptide linkers (spacer peptides). Among the suitable peptide linkers are those described in U.S. Patents 4,751,180 and 4,935,233 or WO 88/09344.
Another method for preparing oligomeric antibody constuct derivatives involves use of a leucine zipper. Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al., 1994, FEBS Letters 344:191, hereby incorporated by reference. The use of a modified leucine zipper that allows for stable trimerization of a heterologous protein fused thereto is described in Fanslow et al., 1994, Semin. Immunol. 6:267-78. In one approach, recombinant fusion proteins comprising elected cell surface molecule antibody fragment or derivative fused to a leucine zipper peptide are expressed in suitable host cells, and the soluble oligomeric elected cell surface molecule antibody fragments or derivatives that form are recovered from the culture supernatant. Covalent modifications of antigen binding proteins are included within the scope of this invention, and are generally, but not always, done post-translationally. For example, several types of covalent modifications of the antigen binding protein are introduced into the molecule by reacting specific amino acid residues of the antigen binding protein with an organic derivatizing agent that is capable of reacting with selected side chains or the N- or C-terminal residues.
Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.
Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.
Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.
Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine epsilon-amino group.
The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.
Carboxyl side groups (aspartyl or glutamyl) are selectively modified by reaction with carbodiimides (R'-N=C=N--R'), where R and R' are optionally different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.
Derivatization with bifunctional agents is useful for crosslinking antigen binding proteins to a water-insoluble support matrix or surface for use in a variety of methods. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are employed for protein immobilization.
Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.
Other modifications include hydroxylation of proline and lysine, phosphorylation of hydroxyl groups of seryl or threonyl residues, methylation of the α-amino groups of lysine, arginine, and histidine side chains (T. E. Creighton, Proteins: Structure and Molecular Properties, W. H. Freeman & Co., San Francisco, 1983, pp. 79-86), acetylation of the N-terminal amine, and amidation of any C-terminal carboxyl group.
Another type of covalent modification of the antigen binding protein included within the scope of this invention comprises altering the glycosylation pattern of the protein. As is known in the art, glycosylation patterns can depend on both the sequence of the protein (e.g., the presence or absence of particular glycosylation amino acid residues, discussed below), or the host cell or organism in which the protein is produced. Particular expression systems are discussed below. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.
Addition of glycosylation sites to the antigen binding protein is conveniently accomplished by altering the amino acid sequence such that it contains one or more of the above-described tripeptide sequences (for N-linked glycosylation sites). The alteration may also be made by the addition of, or substitution by, one or more serine or threonine residues to the starting sequence (for O-linked glycosylation sites). For ease, the antigen binding protein amino acid sequence is preferably altered through changes at the DNA level, particularly by mutating the DNA encoding the target polypeptide at preselected bases such that codons are generated that will translate into the desired amino acids.
Another means of increasing the number of carbohydrate moieties on the antigen binding protein is by chemical or enzymatic coupling of glycosides to the protein. These procedures are advantageous in that they do not require production of the protein in a host cell that has glycosylation capabilities for N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published Sep. 11, 1987, and in Aplin and Wriston, 1981, CRC Crit. Rev. Biochem., pp. 259-306.
Removal of carbohydrate moieties present on the starting antigen binding protein may be accomplished chemically or enzymatically. Chemical deglycosylation requires exposure of the protein to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al., 1987, Arch. Biochem. Biophys. 259:52 and by Edge et al., 1981, Anal. Biochem. 118:131. Enzymatic cleavage of carbohydrate moieties on polypeptides can be achieved by the use of a variety of endo- and exo-glycosidases as described by Thotakura et al., 1987, Meth. Enzymol. 138:350. Glycosylation at potential glycosylation sites may be prevented by the use of the compound tunicamycin as described by Duskin et al., 1982, J. Biol. Chem. 257:3105. Tunicamycin blocks the formation of protein-N-glycoside linkages.
Another type of covalent modification of the antigen binding protein comprises linking the antigen binding protein to various non-proteinaceous polymers, including, but not limited to, various polyols such as polyethylene glycol, polypropylene glycol or polyoxyalkylenes, in the manner set forth in U.S. Pat. Nos. 4,640,835; 4,496,689; 4,301,144; 4,670,417; 4,791,192 or 4,179,337. In addition, as is known in the art, amino acid substitutions may be made in various positions within the antigen binding protein to facilitate the addition of polymers such as PEG.
In some embodiments, the covalent modification of the antigen binding proteins of the invention comprises the addition of one or more labels.
The term "labelling group" means any detectable label. Examples of suitable labelling groups include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by a secondary reporter (e.g., leucine zipper pair sequences, binding sites for secondary antibodies, metal binding domains, epitope tags). In some embodiments, the labelling group is coupled to the antigen binding protein *via* spacer arms of various lengths to reduce potential steric hindrance. Various methods for labelling proteins are known in the art and may be used in performing the present invention.
In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.). In some embodiments, the labelling group is coupled to the antigen binding protein *via* spacer arms of various lengths to reduce potential steric hindrance. Various methods for labelling proteins are known in the art and may be used in performing the present invention. Specific labels include optical dyes, including, but not limited to, chromophores, phosphors and fluorophores, with the latter being specific in many instances. Fluorophores can be either "small molecule" fluores, or proteinaceous fluores.
By "fluorescent label" is meant any molecule that may be detected *via* its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE)
(Molecular Probes, Eugene, OR), FITC, Rhodamine, and Texas Red (Pierce, Rockford, IL), Cy5, Cy5.5, Cy7 (Amersham Life Science, Pittsburgh, PA). Suitable optical dyes, including fluorophores, are described in Molecular Probes Handbook by Richard P. Haugland, hereby expressly incorporated by reference.
Suitable proteinaceous fluorescent labels also include, but are not limited to, green fluorescent protein, including a Renilla, Ptilosarcus, or Aequorea species of GFP (Chalfie et al., 1994, Science 263:802-805), EGFP (Clontech Laboratories, Inc., Genbank Accession Number U55762), blue fluorescent protein (BFP, Quantum Biotechnologies, Inc. 1801 de Maisonneuve Blvd. West, 8th Floor, Montreal, Quebec, Canada H3H 1J9; Stauber, 1998, Biotechniques 24:462-471; Heim et al., 1996, Curr. Biol. 6:178-182), enhanced yellow fluorescent protein (EYFP, Clontech Laboratories, Inc.), luciferase (Ichiki et al., 1993, J. Immunol. 150:5408-5417), β galactosidase (Nolan et al., 1988, Proc. Natl. Acad. Sci. U.S.A. 85:2603-2607) and Renilla (WO92/15673, WO95/07463, WO98/14605, WO98/26277, WO99/49019, U.S. Patent Nos. 5292658, 5418155, 5683888, 5741668, 5777079, 5804387, 5874304, 5876995, 5925558). All of the above-cited references are expressly incorporated herein by reference.

In one embodiment the binding molecule of the invention is characterized in a way that the three domains are consecutively on one polypeptide chain in the order from the N-terminus to the C-terminus
- the first binding domain;
- the second binding domain; and
- the third binding domain.

The invention also provides a single chain binding molecule comprising at least three binding domains comprised in one polypeptide chain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex,
wherein the yield of expressible monomeric binding molecule in relation to the total amount of binding molecule isolated from the culture supernatant of host cells producing the binding molecule depends on the order of the first and second binding domain in said binding molecule.

The invention further provides a single chain binding molecule comprising at least three binding domains comprised in one polypeptide chain in the order first domain, second domain and third domain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex;
wherein the yield of monomeric binding molecule isolated from the culture supernatant of host cells producing the binding molecule is at least 1.5 times higher than the yield of monomeric binding molecule isolated from the culture supernatant of host cells producing a binding molecule comprising the binding domain capable of binding to serum albumin is at the C-terminus of the molecule.
It is further preferred that this yield of monomeric binding molecules is 2 fold higher, more preferably, 2.5 fold higher, even more preferred 3 fold, 4 fold or 5 fold higher.

In one embodiment the binding molecule of the invention is characterized in a way that at least one of the binding domains, preferably the second and/or third binding domain, is an scFv or single domain antibody.

Also in one embodiment of the binding molecule of the invention the molecule comprises one or more further heterologous polypeptide.

A binding molecule of the invention may also comprise a His-tag as a heterologus poypeptide. It is preferred for the binding molecule of the invention that the His-tag is positioned at the C-terminus of the third binding domain.

The binding molecule of the invention may also comprise additional domains, which e.g. are helpful in the isolation of the molecule or relate to an adapted pharmacokinetic profile of the molecule.
Domains helpful for the isolation of a binding molecule may be elected from peptide motives or secondarily introduced moieties, which can be captured in an isolation method, e.g. an isolation column. A non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. Strepll-tag) and His-tag. All herein disclosed binding molecules characterized by the identified CDRs are preferred to comprise a His-tag domain, which is generally known as a repeat of consecutive His residues in the amino acid sequence of a molecule, preferably of six His residues.
As apparent from the appended examples, it appears that the yield of monomeric binding molecule of the invention isolated from the cell supernatant of host cells expressing the binding molecule may be further increased by using binding molecules, which do not comprise a His-tag. Thus, without being bound by theory, the electing a binding molecule of the invention, which does not comprise a His-tag domain can result in further increasing the yield of monomeric binding molecules from the cell supernatant of host cells expressing the binding molecule. Accordingly, binding molecules which do not comprise a His-tag are alternatively preferred.

The invention also provides a binding molecule, wherein
(a) the first binding domain is capable of binding to human and non-human primate serum albumin;
(b) the second binding domain is capable of binding to the cell surface molecule on a human and a non-human primate cell, and
(c) the third binding domain is capable of binding to the T cell CD3 receptor complex on a human and a non-human primate cell.

In one embodiment the binding molecule according to the invention is characterized that the first binding domain capable of binding to serum albumin is derived from a combinatorial library or an antibody binding domain.

In a preferred embodiment of the binding molecule of the invention the first binding domain comprises between 10 and 25 aa residues.

In one embodiment of the binding molecule of the invention the first binding domain capable of binding to serum albumin comprises the amino acid sequence Asp-Xaa-Cys-Leu-Pro-Xaa-Trp-Gly-Cys-Leu-Trp, wherein Xaa is any amino acid.

In one embodiment of the binding molecule of the invention the first binding domain capable of binding to serum albumin is derived from a CDR of a single domain antibody.

Also in one embodiment of the binding molecule of the invention the first binding domain is binding to serum albumin with an affinity (KD) of ≤ 500 nM.

In one embodiment of the binding molecule of the invention
- the second binding domain is binding to the cell surface molecule on a target cell with an affinity (KD) of ≤ 100 nM; and
- the third binding domain is binding to the T cell CD3 receptor complex with an affinity (KD) of ≤ 100nM.

In one embodiment of the binding molecule of the invention the binding molecule shows cytotoxic activity in an in vitro assay measuring the lysis of target cells by effector cells in the presence of 10% human serum albumin.

In a preferred embodiment of the binding molecule of the invention the molecule consists of a single polypeptide chain.

In one embodiment of the binding molecule of the invention
(a) the second binding domain comprises an antibody derived VL and VH chain; and/or
(b) the third binding domain comprises an antibody derived VL and VH chain.

Also in one embodiment of the binding molecule of the invention the molecule comprises one or more further heterologous polypeptide.

In one embodiment of the binding molecule of the invention the first binding domain capable of binding to a cell surface molecule is binding to a tumor antigen.

In a preferred embodiment of the binding molecule of the invention the third binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 WO 2008/119567 and comprises at least the amino acid sequence Gln-Asp-Gly-Asn-Glu.

In one aspect of the invention, the third binding domain is capable of binding to to human CD3 and to macaque CD3, preferably to human CD3 epsilon and to macaque CD3 epsilon. Additionally or alternatively, the third binding domain is capable of binding to *Callithrix jacchus, Saguinus oedipus* and/or *Saimiri sciureus* CD3 epsilon. According to these embodiments, one or both binding domains of the binding molecule of the invention are preferably cross-species specific for members of the mammalian order of primates. Cross-species specific CD3 binding domains are, for example, described in WO 2008/119567.

It is particularly preferred for the binding molecule of the present invention that the third binding domain capable of binding to the T cell CD3 receptor complex comprises a VL region comprising CDR-L1, CDR-L2 and CDR-L3 selected from:
(a) CDR-L1 as depicted in SEQ ID NO: 27 of WO 2008/119567, CDR-L2 as depicted in SEQ ID NO: 28 of WO 2008/119567 and CDR-L3 as depicted in SEQ ID NO: 29 of WO 2008/119567;
(b) CDR-L1 as depicted in SEQ ID NO: 117 of WO 2008/119567, CDR-L2 as depicted in SEQ ID NO: 118 of WO 2008/119567 and CDR-L3 as depicted in SEQ ID NO: 119 of WO 2008/119567; and
(c) CDR-L1 as depicted in SEQ ID NO: 153 of WO 2008/119567, CDR-L2 as depicted in SEQ ID NO: 154 of WO 2008/119567 and CDR-L3 as depicted in SEQ ID NO: 155 of WO 2008/119567.

In an alternatively preferred embodiment of the binding molecule of the present invention, the third binding domain capable of binding to the T cell CD3 receptor complex comprises a VH region comprising CDR-H 1, CDR-H2 and CDR-H3 selected from:
(a) CDR-H1 as depicted in SEQ ID NO: 12 of WO 2008/119567, CDR-H2 as depicted in SEQ ID NO: 13 of WO 2008/119567 and CDR-H3 as depicted in SEQ ID NO: 14 of WO 2008/119567;
(b) CDR-H1 as depicted in SEQ ID NO: 30 of WO 2008/119567, CDR-H2 as depicted in SEQ ID NO: 31 of WO 2008/119567 and CDR-H3 as depicted in SEQ ID NO: 32 of WO 2008/119567;
(c) CDR-H1 as depicted in SEQ ID NO: 48 of WO 2008/119567, CDR-H2 as depicted in SEQ ID NO: 49 of WO 2008/119567 and CDR-H3 as depicted in SEQ ID NO: 50 of WO 2008/119567;
(d) CDR-H1 as depicted in SEQ ID NO: 66 of WO 2008/119567, CDR-H2 as depicted in SEQ ID NO: 67 of WO 2008/119567 and CDR-H3 as depicted in SEQ ID NO: 68 of WO 2008/119567;
(e) CDR-H1 as depicted in SEQ ID NO: 84 of WO 2008/119567, CDR-H2 as depicted in SEQ ID NO: 85 of WO 2008/119567 and CDR-H3 as depicted in SEQ ID NO: 86 of WO 2008/119567;
(f) CDR-H1 as depicted in SEQ ID NO: 102 of WO 2008/119567, CDR-H2 as depicted in SEQ ID NO: 103 of WO 2008/119567 and CDR-H3 as depicted in SEQ ID NO: 104 of WO 2008/119567;
(g) CDR-H1 as depicted in SEQ ID NO: 120 of WO 2008/119567, CDR-H2 as depicted in SEQ ID NO: 121 of WO 2008/119567 and CDR-H3 as depicted in SEQ ID NO: 122 of WO 2008/119567;
(h) CDR-H1 as depicted in SEQ ID NO: 138 of WO 2008/119567, CDR-H2 as depicted in SEQ ID NO: 139 of WO 2008/119567 and CDR-H3 as depicted in SEQ ID NO: 140 of WO 2008/119567;
(i) CDR-H1 as depicted in SEQ ID NO: 156 of WO 2008/119567, CDR-H2 as depicted in SEQ ID NO: 157 of WO 2008/119567 and CDR-H3 as depicted in SEQ ID NO: 158 of WO 2008/119567; and
(j) CDR-H1 as depicted in SEQ ID NO: 174 of WO 2008/119567, CDR-H2 as depicted in SEQ ID NO: 175 of WO 2008/119567 and CDR-H3 as depicted in SEQ ID NO: 176 of WO 2008/119567.

It is further preferred for the binding molecule of the present invention that the third binding domain capable of binding to the T cell CD3 receptor complex comprises a VL region selected from the group consisting of a VL region as depicted in SEQ ID NO: 35, 39, 125, 129, 161 or 165 of WO 2008/119567.

It is alternatively preferred that the third binding domain capable of binding to the T cell CD3 receptor complex comprises a VH region selected from the group consisting of a VH region as depicted in SEQ ID NO: 15, 19, 33, 37, 51, 55, 69, 73, 87, 91, 105, 109, 123, 127, 141, 145, 159, 163, 177 or 181 of WO 2008/119567.

More preferably, the binding molecule of the present invention is characterized by the third binding domain capable of binding to the T cell CD3 receptor complex comprising a VL region and a VH region selected from the group consisting of:
(a) a VL region as depicted in SEQ ID NO: 17 or 21 of WO 2008/119567 and a VH region as depicted in SEQ ID NO: 15 or 19 of WO 2008/119567;
(b) a VL region as depicted in SEQ ID NO: 35 or 39 of WO 2008/119567 and a VH region as depicted in SEQ ID NO: 33 or 37 of WO 2008/119567;
(c) a VL region as depicted in SEQ ID NO: 53 or 57 of WO 2008/119567 and a VH region as depicted in SEQ ID NO: 51 or 55 of WO 2008/119567;
(d) a VL region as depicted in SEQ ID NO: 71 or 75 of WO 2008/119567 and a VH region as depicted in SEQ ID NO: 69 or 73 of WO 2008/119567;
(e) a VL region as depicted in SEQ ID NO: 89 or 93 of WO 2008/119567 and a VH region as depicted in SEQ ID NO: 87 or 91 of WO 2008/119567;
(f) a VL region as depicted in SEQ ID NO: 107 or 111 of WO 2008/119567 and a VH region as depicted in SEQ ID NO: 105 or 109 of WO 2008/119567;
(g) a VL region as depicted in SEQ ID NO: 125 or 129 of WO 2008/119567 and a VH region as depicted in SEQ ID NO: 123 or 127 of WO 2008/119567;
(h) a VL region as depicted in SEQ ID NO: 143 or 147 of WO 2008/119567 and a VH region as depicted in SEQ ID NO: 141 or 145 of WO 2008/119567;
(i) a VL region as depicted in SEQ ID NO: 161 or 165 of WO 2008/119567 and a VH region as depicted in SEQ ID NO: 159 or 163 of WO 2008/119567; and
(j) a VL region as depicted in SEQ ID NO: 179 or 183 of WO 2008/119567 and a VH region as depicted in SEQ ID NO: 177 or 181 of WO 2008/119567.

According to a preferred embodiment of the binding molecule of the present invention, in particular the third binding domain capable of binding to the T cell CD3 receptor complex, the pairs of VH-regions and VL-regions are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence.

A preferred embodiment of the above described binding molecule of the present invention is characterized by the third binding domain capable of binding to the T cell CD3 receptor complex comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187 of WO 2008/119567.

In one embodiment the binding molecule of the invention is characterized by an amino acid sequence as depicted in SEQ ID NOs: 8, 12, 16, 20, 24, 26, 30, or 34.

An alternative embodiment of the invention provides a method for the production of binding molecule of the invention, the method comprising the step of:
- selecting for binding molecules comprising a binding domain, which is capable of binding to a cell surface molecule on a target cell, comprising at the N-terminus a binding domain which is capable of binding to serum albumin.

In one embodiment the method of the invention further comprises the step of:
- adding to the molecule an additional binding domain, which is capable of binding to the T cell CD3 receptor complex.

The invention further provides a nucleic acid molecule having a sequence encoding a binding molecule of the invention.

Furthermore, the invention provides a vector comprising a nucleic acid sequence of the invention. Moreover, the invention provides a host cell transformed or transfected with the nucleic acid sequence of the invention.

In one embodiment the invention provides a process for the production of a binding molecule of the invention or produced by a method of the invention, said process comprising culturing a host cell of the invention under conditions allowing the expression of the binding molecule a of the invention or produced by a method of the invention and recovering the produced binding molecule from the culture.

Moreover, the invention provides a pharmaceutical composition comprising a binding molecule of the invention or produced according to the process of the invention

The formulations described herein are useful as pharmaceutical compositions in the treatment, amelioration and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.
Those "in need of treatment" include those already with the disorder, as well as those in which the disorder is to be prevented. The term "disease" is any condition that would benefit from treatment with the protein formulation described herein. This includes chronic and acute disorders or diseases including those pathological conditions that predispose the mammal to the disease in question. Non-limiting examples of diseases/disorders to be treated herein include proliferative disease, a tumorous disease, or an immunological disorder.

In some embodiments, the invention provides a pharmaceutical composition comprising a therapeutically effective amount of one or a plurality of the binding molecule of the invention together with a pharmaceutically effective diluents, carrier, solubilizer, emulsifier, preservative, and/or adjuvant. Pharmaceutical compositions of the invention include, but are not limited to, liquid, frozen, and lyophilized compositions.
Preferably, formulation materials are nontoxic to recipients at the dosages and concentrations employed. In specific embodiments, pharmaceutical compositions comprising a therapeutically effective amount of an binding molecule of the invention.
In certain embodiments, the pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine, proline, or lysine); antimicrobials; antioxidants (such as ascorbic acid, sodium sulfite or sodium hydrogen-sulfite); buffers (such as borate, bicarbonate, Tris-HCI, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, REMINGTON'S PHARMACEUTICAL SCIENCES, 18" Edition, (A. R. Genrmo, ed.), 1990, Mack Publishing Company.

In certain embodiments, the optimal pharmaceutical composition will be determined by one skilled in the art depending upon, for example, the intended route of administration, delivery format and desired dosage. See, for example, REMINGTON'S PHARMACEUTICAL SCIENCES, supra. In certain embodiments, such compositions may influence the physical state, stability, rate of in vivo release and rate of in vivo clearance of the antigen binding proteins of the invention. In certain embodiments, the primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefore. In certain embodiments of the invention, human antibody or antigen binding fragment thereof of the invention or the binding molecule of the invention compositions may be prepared for storage by mixing the selected composition having the desired degree of purity with optional formulation agents (REMINGTON'S PHARMACEUTICAL SCIENCES, supra) in the form of a lyophilized cake or an aqueous solution. Further, in certain embodiments, the human antibody or antigen binding fragment thereof of the invention or the binding molecule of the invention may be formulated as a lyophilizate using appropriate excipients such as sucrose.
The pharmaceutical compositions of the invention can be selected for parenteral delivery. Alternatively, the compositions may be selected for inhalation or for delivery through the digestive tract, such as orally. Preparation of such pharmaceutically acceptable compositions is within the skill of the art. The formulation components are present preferably in concentrations that are acceptable to the site of administration. In certain embodiments, buffers are used to maintain the composition at physiological pH or at a slightly lower pH, typically within a pH range of from about 5 to about 8.
When parenteral administration is contemplated, the therapeutic compositions for use in this invention may be provided in the form of a pyrogen-free, parenterally acceptable aqueous solution comprising the desired human antibody or antigen binding fragment thereof of the invention or the binding molecule of the invention in a pharmaceutically acceptable vehicle. A particularly suitable vehicle for parenteral injection is sterile distilled water in which the binding molecule of the invention is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product which can be delivered via depot injection. In certain embodiments, hyaluronic acid may also be used, having the effect of promoting sustained duration in the circulation. In certain embodiments, implantable drug delivery devices may be used to introduce the desired antigen binding protein.

Additional pharmaceutical compositions will be evident to those skilled in the art, including formulations involving the binding molecule of the invention in sustained- or controlled-delivery formulations. Techniques for formulating a variety of other sustained- or controlled-delivery means, such as liposome carriers, bio-erodible microparticles or porous beads and depot injections, are also known to those skilled in the art. See, for example, International Patent Application No. PCT/US93/00829, which is incorporated by reference and describes controlled release of porous polymeric microparticles for delivery of pharmaceutical compositions. Sustained-release preparations may include semipermeable polymer matrices in the form of shaped articles, e.g., films, or microcapsules. Sustained release matrices may include polyesters, hydrogels, polylactides (as disclosed in U.S. Pat. No. 3,773,919 and European Patent Application Publication No. EP 058481, each of which is incorporated by reference), copolymers of L-glutamic acid and gamma ethyl-L-glutamate (Sidman et al., 1983, Biopolymers 2:547-556), poly (2-hydroxyethyl-methacrylate) (Langer et al., 1981, J. Biomed. Mater. Res. 15:167-277 and Langer, 1982, Chem. Tech. 12:98-105), ethylene vinyl acetate (Langer et al., 1981, supra) or poly-D(-)-3-hydroxybutyric acid (European Patent Application Publication No. EP 133,988). Sustained release compositions may also include liposomes that can be prepared by any of several methods known in the art. See, e.g., Eppstein et al., 1985, Proc. Natl. Acad. Sci. U.S.A. 82:3688-3692; European Patent Application Publication Nos. EP 036,676; EP 088,046 and EP 143,949, incorporated by reference.
Pharmaceutical compositions used for in vivo administration are typically provided as sterile preparations. Sterilization can be accomplished by filtration through sterile filtration membranes. When the composition is lyophilized, sterilization using this method may be conducted either prior to or following lyophilization and reconstitution. Compositions for parenteral administration can be stored in lyophilized form or in a solution. Parenteral compositions generally are placed into a container having a sterile access port, for example, an intravenous solution bag or vial having a stopper pierceable by a hypodermic injection needle.
Aspects of the invention includes self-buffering binding molecule of the invention formulations, which can be used as pharmaceutical compositions, as described in international patent application WO 06138181A2 (PCT/US2006/022599), which is incorporated by reference in its entirety herein.
As discussed above, certain embodiments provide binding molecule of the invention protein compositions, particularly pharmaceutical compositions of the invention, that comprise, in addition to the binding molecule of the invention, one or more excipients such as those illustratively described in this section and elsewhere herein. Excipients can be used in the invention in this regard for a wide variety of purposes, such as adjusting physical, chemical, or biological properties of formulations, such as adjustment of viscosity, and or processes of the invention to improve effectiveness and or to stabilize such formulations and processes against degradation and spoilage due to, for instance, stresses that occur during manufacturing, shipping, storage, pre-use preparation, administration, and thereafter.
A variety of expositions are available on protein stabilization and formulation materials and methods useful in this regard, such as Arakawa et al., "Solvent interactions in pharmaceutical formulations," Pharm Res. 8(3): 285-91 (1991); Kendrick et al., "Physical stabilization of proteins in aqueous solution," in: RATIONAL DESIGN OF STABLE PROTEIN FORMULATIONS: THEORY AND PRACTICE, Carpenter and Manning, eds. Pharmaceutical Biotechnology. 13: 61-84 (2002), and Randolph et al., "Surfactant-protein interactions," Pharm Biotechnol. 13: 159-75 (2002), each of which is herein incorporated by reference in its entirety, particularly in parts pertinent to excipients and processes of the same for self-buffering protein formulations in accordance with the current invention, especially as to protein pharmaceutical products and processes for veterinary and/or human medical uses.
Salts may be used in accordance with certain embodiments of the invention to, for example, adjust the ionic strength and/or the isotonicity of a formulation and/or to improve the solubility and/or physical stability of a protein or other ingredient of a composition in accordance with the invention.
As is well known, ions can stabilize the native state of proteins by binding to charged residues on the protein's surface and by shielding charged and polar groups in the protein and reducing the strength of their electrostatic interactions, attractive, and repulsive interactions. Ions also can stabilize the denatured state of a protein by binding to, in particular, the denatured peptide linkages (--CONH) of the protein. Furthermore, ionic interaction with charged and polar groups in a protein also can reduce intermolecular electrostatic interactions and, thereby, prevent or reduce protein aggregation and insolubility.
Ionic species differ significantly in their effects on proteins. A number of categorical rankings of ions and their effects on proteins have been developed that can be used in formulating pharmaceutical compositions in accordance with the invention. One example is the Hofmeister series, which ranks ionic and polar non-ionic solutes by their effect on the conformational stability of proteins in solution. Stabilizing solutes are referred to as "kosmotropic." Destabilizing solutes are referred to as "chaotropic." Kosmotropes commonly are used at high concentrations (e.g., >1 molar ammonium sulfate) to precipitate proteins from solution ("salting-out"). Chaotropes commonly are used to denture and/or to solubilize proteins ("salting-in"). The relative effectiveness of ions to "salt-in" and "salt-out" defines their position in the Hofmeister series.
Free amino acids can be used in the binding molecule of the invention formulations in accordance with various embodiments of the invention as bulking agents, stabilizers, and antioxidants, as well as other standard uses. Lysine, proline, serine, and alanine can be used for stabilizing proteins in a formulation. Glycine is useful in lyophilization to ensure correct cake structure and properties. Arginine may be useful to inhibit protein aggregation, in both liquid and lyophilized formulations. Methionine is useful as an antioxidant.
Polyols include sugars, e.g., mannitol, sucrose, and sorbitol and polyhydric alcohols such as, for instance, glycerol and propylene glycol, and, for purposes of discussion herein, polyethylene glycol (PEG) and related substances. Polyols are kosmotropic. They are useful stabilizing agents in both liquid and lyophilized formulations to protect proteins from physical and chemical degradation processes. Polyols also are useful for adjusting the tonicity of formulations. Among polyols useful in select embodiments of the invention is mannitol, commonly used to ensure structural stability of the cake in lyophilized formulations. It ensures structural stability to the cake. It is generally used with a lyoprotectant, e.g., sucrose. Sorbitol and sucrose are among preferred agents for adjusting tonicity and as stabilizers to protect against freeze-thaw stresses during transport or the preparation of bulks during the manufacturing process. Reducing sugars (which contain free aldehyde or ketone groups), such as glucose and lactose, can glycate surface lysine and arginine residues. Therefore, they generally are not among preferred polyols for use in accordance with the invention. In addition, sugars that form such reactive species, such as sucrose, which is hydrolyzed to fructose and glucose under acidic conditions, and consequently engenders glycation, also is not among preferred polyols of the invention in this regard. PEG is useful to stabilize proteins and as a cryoprotectant and can be used in the invention in this regard.
Embodiments of the binding molecule of the invention formulations further comprise surfactants. Protein molecules may be susceptible to adsorption on surfaces and to denaturation and consequent aggregation at air-liquid, solid-liquid, and liquid-liquid interfaces. These effects generally scale inversely with protein concentration. These deleterious interactions generally scale inversely with protein concentration and typically are exacerbated by physical agitation, such as that generated during the shipping and handling of a product.
Surfactants routinely are used to prevent, minimize, or reduce surface adsorption. Useful surfactants in the invention in this regard include polysorbate 20, polysorbate 80, other fatty acid esters of sorbitan polyethoxylates, and poloxamer 188.
Surfactants also are commonly used to control protein conformational stability. The use of surfactants in this regard is protein-specific since, any given surfactant typically will stabilize some proteins and destabilize others.
Polysorbates are susceptible to oxidative degradation and often, as supplied, contain sufficient quantities of peroxides to cause oxidation of protein residue side-chains, especially methionine. Consequently, polysorbates should be used carefully, and when used, should be employed at their lowest effective concentration. In this regard, polysorbates exemplify the general rule that excipients should be used in their lowest effective concentrations.
Embodiments of the binding molecule of the invention formulations further comprise one or more antioxidants. To some extent deleterious oxidation of proteins can be prevented in pharmaceutical formulations by maintaining proper levels of ambient oxygen and temperature and by avoiding exposure to light. Antioxidant excipients can be used as well to prevent oxidative degradation of proteins. Among useful antioxidants in this regard are reducing agents, oxygen/free-radical scavengers, and chelating agents. Antioxidants for use in therapeutic protein formulations in accordance with the invention preferably are water-soluble and maintain their activity throughout the shelf life of a product. EDTA is a preferred antioxidant in accordance with the invention in this regard.
Antioxidants can damage proteins. For instance, reducing agents, such as glutathione in particular, can disrupt intramolecular disulfide linkages. Thus, antioxidants for use in the invention are selected to, among other things, eliminate or sufficiently reduce the possibility of themselves damaging proteins in the formulation.
Formulations in accordance with the invention may include metal ions that are protein co-factors and that are necessary to form protein coordination complexes, such as zinc necessary to form certain insulin suspensions. Metal ions also can inhibit some processes that degrade proteins. However, metal ions also catalyze physical and chemical processes that degrade proteins. Magnesium ions (10-120 mM) can be used to inhibit isomerization of aspartic acid to isoaspartic acid. Ca+2 ions (up to 100 mM) can increase the stability of human deoxyribonuclease. Mg+2, Mn+2, and Zn+2, however, can destabilize rhDNase. Similarly, Ca+2 and Sr+2 can stabilize Factor VIII, it can be destabilized by Mg+2, Mn+2 and Zn+2, Cu+2 and Fe+2, and its aggregation can be increased by AI+3 ions.

Embodiments of the binding molecule of the invention formulations further comprise one or more preservatives. Preservatives are necessary when developing multi-dose parenteral formulations that involve more than one extraction from the same container. Their primary function is to inhibit microbial growth and ensure product sterility throughout the shelf-life or term of use of the drug product. Commonly used preservatives include benzyl alcohol, phenol and m-cresol. Although preservatives have a long history of use with small-molecule parenterals, the development of protein formulations that includes preservatives can be challenging. Preservatives almost always have a destabilizing effect (aggregation) on proteins, and this has become a major factor in limiting their use in multi-dose protein formulations. To date, most protein drugs have been formulated for single-use only. However, when multi-dose formulations are possible, they have the added advantage of enabling patient convenience, and increased marketability. A good example is that of human growth hormone (hGH) where the development of preserved formulations has led to commercialization of more convenient, multi-use injection pen presentations. At least four such pen devices containing preserved formulations of hGH are currently available on the market. Norditropin (liquid, Novo Nordisk), Nutropin AQ (liquid, Genentech) & Genotropin (lyophilized--dual chamber cartridge, Pharmacia & Upjohn) contain phenol while Somatrope (Eli Lilly) is formulated with m-cresol. Several aspects need to be considered during the formulation and development of preserved dosage forms. The effective preservative concentration in the drug product must be optimized. This requires testing a given preservative in the dosage form with concentration ranges that confer anti-microbial effectiveness without compromising protein stability.
As might be expected, development of liquid formulations containing preservatives are more challenging than lyophilized formulations. Freeze-dried products can be lyophilized without the preservative and reconstituted with a preservative containing diluent at the time of use. This shortens the time for which a preservative is in contact with the protein, significantly minimizing the associated stability risks. With liquid formulations, preservative effectiveness and stability should be maintained over the entire product shelf-life (about 18 to 24 months). An important point to note is that preservative effectiveness should be demonstrated in the final formulation containing the active drug and all excipient components.
The binding molecule of the invention generally will be designed for specific routes and methods of administration, for specific administration dosages and frequencies of administration, for specific treatments of specific diseases, with ranges of bio-availability and persistence, among other things. Formulations thus may be designed in accordance with the invention for delivery by any suitable route, including but not limited to orally, aurally, opthalmically, rectally, and vaginally, and by parenteral routes, including intravenous and intraarterial injection, intramuscular injection, and subcutaneous injection.
Once the pharmaceutical composition has been formulated, it may be stored in sterile vials as a solution, suspension, gel, emulsion, solid, crystal, or as a dehydrated or lyophilized powder. Such formulations may be stored either in a ready-to-use form or in a form (e.g., lyophilized) that is reconstituted prior to administration. The invention also provides kits for producing a single-dose administration unit. The kits of the invention may each contain both a first container having a dried protein and a second container having an aqueous formulation. In certain embodiments of this invention, kits containing single and multi-chambered pre-filled syringes (e.g., liquid syringes and lyosyringes) are provided. The therapeutically effective amount of an binding molecule of the invention protein-containing pharmaceutical composition to be employed will depend, for example, upon the therapeutic context and objectives. One skilled in the art will appreciate that the appropriate dosage levels for treatment will vary depending, in part, upon the molecule delivered, the indication for which the binding molecule of the invention is being used, the route of administration, and the size (body weight, body surface or organ size) and/or condition (the age and general health) of the patient. In certain embodiments, the clinician may titer the dosage and modify the route of administration to obtain the optimal therapeutic effect. A typical dosage may range from about 0.1 µg/kg to up to about 30 mg/kg or more, depending on the factors mentioned above. In specific embodiments, the dosage may range from 1.0 µg/kg up to about 20 mg/kg, optionally from 10 µg/kg up to about 10 mg/kg or from 100 µg/kg up to about 5 mg/kg.
A therapeutic effective amount of an binding molecule of the invention preferably results in a decrease in severity of disease symptoms, in increase in frequency or duration of disease symptom-free periods or a prevention of impairment or disability due to the disease affliction. For treating specific tumors expressing the cell surface molecule bound by the second binding domain of the binding molecule of the invention, a therapeutically effective amount of the binding molecule of the invention, e.g. an anti-cell surface molecule/CD3 binding molecule, preferably inhibits cell growth or tumor growth by at least about 20%, at least about 40%, at least about 50%, at least about 60%, at least about 70%, at least about 80%, or at least about 90% relative to untreated patients. The ability of a compound to inhibit tumor growth may be evaluated in an animal model predictive of efficacy in human tumors.
Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163, all incorporated by reference herein.

According to one embodiment of the invention the binding molecule of the invention, or the binding molecule produced according to a method of the invention is for use in the prevention, treatment or amelioration of a disease selected from the group consisting of a proliferative disease, an inflammatory disease, an infectious disease and an autoimmune disease.

In one embodiment the invention provides a method for the treatment or amelioration of a disease selected from the group consisting of a proliferative disease, an inflammatory disease, an infectious disease and an autoimmune disease, comprising the step of administering to a subject in need thereof the binding molecule of the invention, or the binding molecule produced according to a method of the invention.

Also in one embodiment the invention provides a kit comprising a binding molecule of the invention or produced according to a method of the invention, a nucleic acid molecule of the invention, a vector of the invention, or a host cell of the invention.

It should be understood that the inventions herein are not limited to particular methodology, protocols, or reagents, as such can vary. The discussion and examples provided herein are presented for the purpose of describing particular embodiments only and are not intended to limit the scope of the present invention, which is defined solely by the claims.
All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### Examples:

The following examples are provided for the purpose of illustrating specific embodiments or features of the present invention. These examples should not be construed as to limit the scope of this invention. The examples are included for purposes of illustration, and the present invention is limited only by the claims.

### Example 1 -

### BiTE production and purification:

BiTE antibodies were purified from culture supernatant of stably transfected chinese hamster ovary cells (CHO) adapted to 20 nM MTX. To generate one liter of supernatant for purification of BiTE antibody constructs, the cells were grown in roller bottles at a starting cell density of 5 x 10⁴ cells per ml with nucleoside-free HyQ PF CHO liquid soy medium (with 4.0 mM L-Glutamine and 0.1% Pluronic F-68; HyClone). After color change of the added color indicator phenol red from red to orange the cultivation was extended for two more days before harvesting. The cells were removed by centrifugation and the supernatant containing the expressed protein was stored at -20°C until protein purification.

Äkta® Explorer Systems (GE Life Sciences) controlled by Unicorn® Software were used for chromatography. Immobilized metal affinity chromatography (IMAC) was performed using Fractogel EMD chelate® (Merck, Darmstadt) which was loaded with ZnCl₂ according to the protocol provided by the manufacturer. The column was equilibrated with buffer A (20 mM sodium phosphate buffer pH 7.2, 0.1 M NaCl , 10 mM imidazole) and the cell culture supernatant (1000 ml) applied to the column (10 ml) at a flow rate of 4 ml/min. The column was washed with buffer A to remove unbound sample. Bound protein was eluted using a two step gradient of buffer B (20 mM sodium phosphate buffer, 0.1 M NaCl, 0.5 M imidazole, pH 7.2) according to the following procedure:
Step 1: 10 % buffer B in 5 column volumes
Step 2: 100% buffer B in 5 column volumes

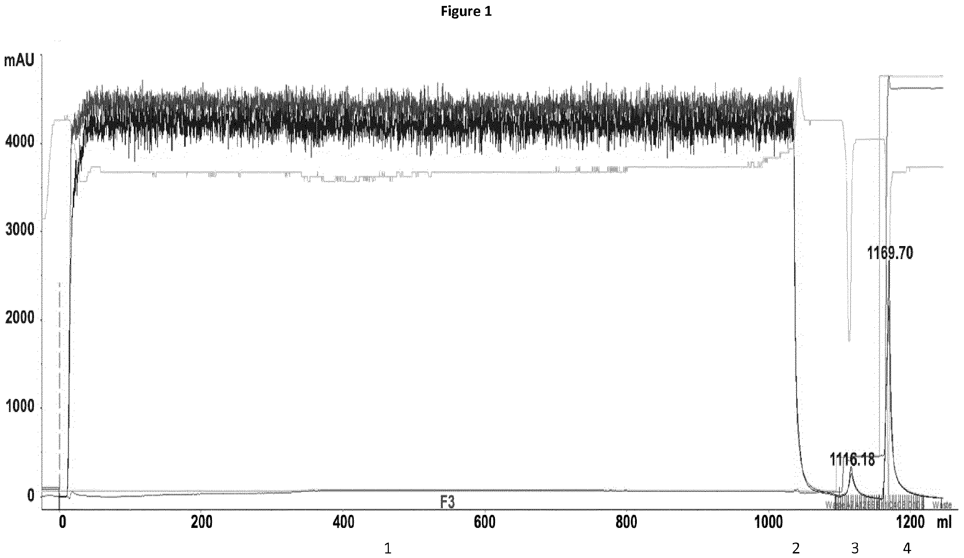

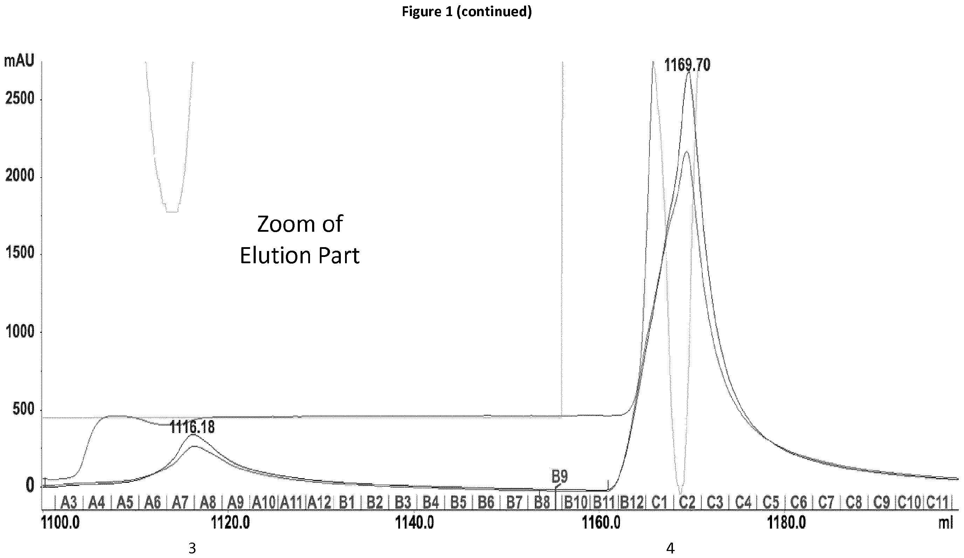

An elution profile from the IMAC purification is exemplarily shown for a SA-21-CEAxCD3 binding molecule in figure 1.

Eluted protein fractions from step 2 were pooled for further purification and concentrated to 3 ml final volume using Vivaspin (Sartorius-Stedim, Gottingen-Germany) centrifugation units with PES membran and a molecular weight cut-off of 10 kDa. All chemicals were of research grade and purchased from Sigma (Deisenhofen, Germany) or Merck (Darmstadt, Germany).

Size exclusion chromatography SEC was performed on a HiLoad 16/60 Superdex 200 prep grade column (GE Lifesciences) equilibrated with SEC buffer (10 mM citric acid, 75 mM lysine-HCl, pH 7.2) at a flow rate of 1 ml/min. BiTE antibody monomer and dimer fractions were pooled and a 24% trehalose stock solution was added to reach a final trehalose concentration of 4%. Eluted protein samples were subjected to reducing SDS-PAGE and Anti His TAG Western Blot for analysis.

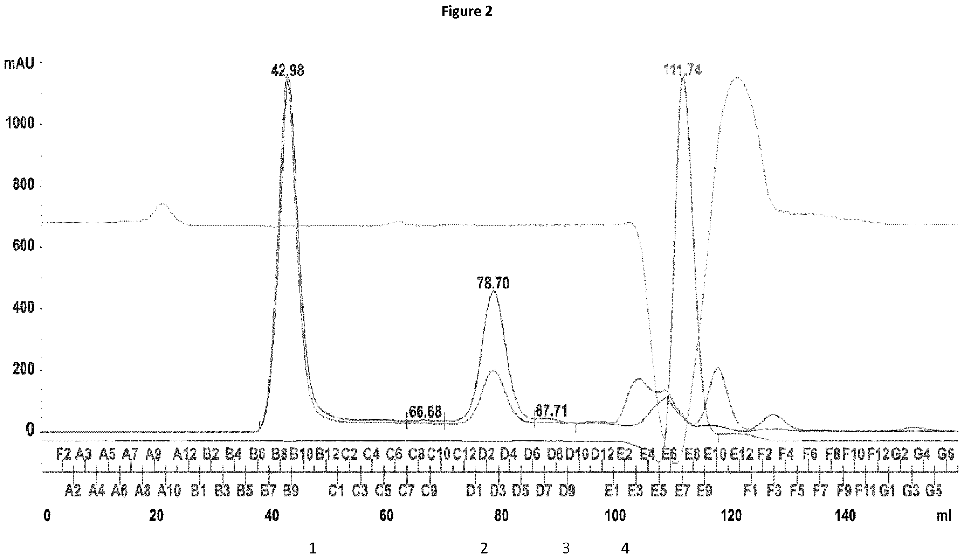

SEC Protein pools (Pool_1 = pooled SEC fractions containing the dimeric BiTE protein isoform, Pool_2 = pooled SEC fractions containing the monomeric BiTE protein) were measured for 280 nm absorption in polycarbonate cuvettes with 1 cm lightpath (Eppendorf, Hamburg-Germany) and protein concentration was calculated on the base of the Vector NTI protein analysis factor for each protein. A SEC profile is exemplarily shown for a SA-21-CEAxCD3 binding molecule in figure 2.

**Table 2: yield of monomeric biding molecules isolated from the culture supernatant of CHO cells stably expressing the identified binding molecules**

| **Binding molecule** | **HSA binding domain** | **Yield [µg/l culture supernatant]** |
|---|---|---|
| CD33 x CD3 | N-term SA21 | 1277 |
| | C-term SA21 | 72 |
| | | |
| CEA x CD3 + His-tag | N-term SA21 | 719 |
| | C-term SA21 | 255 |
| | | |
| CEA x CD3 + His-tag | N-term SA25 | 660 |
| | C-term SA25 | 38 |
| | | |
| CEA x CD3 w/o His-tag | N-term SA21 | 761 |
| | C-term SA21 | 490 |
| | | |
| CEA x CD3 w/o His-tag | N-term SA08 | 1028 |
| | C-term SA08 | 216 |
| | | |
| CEA x CD3 w/o His-tag | N-term SA25 | 981 |
| | C-term SA25 | 450 |

### Example 2 -

### Cytotoxic activity: Chromium release assay with stimulated human T cells

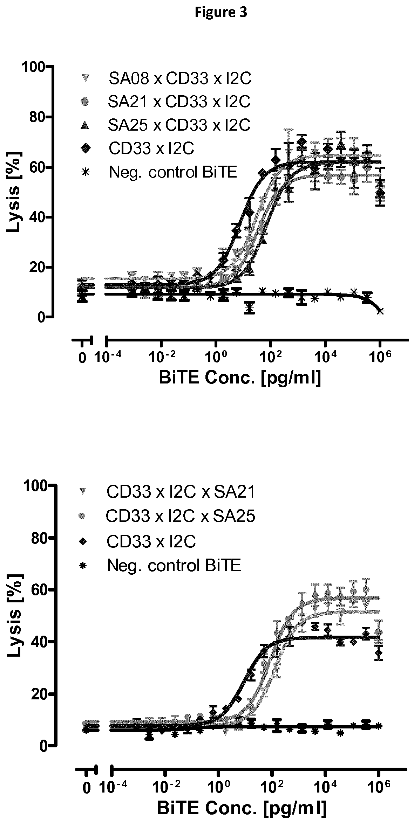

Stimulated T cells enriched for CD8⁺ T cells were obtained as described below:
A petri dish (145 mm diameter, Greiner bio-one GmbH, Kremsmünster) was coated with a commercially available anti-CD3 specific antibody (OKT3, Orthoclone) in a final concentration of 1 µg/ml for 1 hour at 37°C. Unbound protein was removed by one washing step with PBS. 3 - 5 x 10⁷ human PBMC were added to the precoated petri dish in 120 ml of RPMI 1640 with stabilized glutamine / 10% FCS / IL-2 20 U/ml (Proleukin®, Chiron) and stimulated for 2 days. On the third day, the cells were collected and washed once with RPMI 1640. IL-2 was added to a final concentration of 20 U/ml and the cells were cultured again for one day in the same cell culture medium as above.
CD8⁺ cytotoxic T lymphocytes (CTLs) were enriched by depletion of CD4⁺ T cells and CD56⁺ NK cells using Dynal-Beads according to the manufacturer's protocol.
Human CD33-transfected CHO target cells were washed twice with PBS and labeled with 11.1 MBq ⁵¹Cr in a final volume of 100 µl RPMI with 50% FCS for 60 minutes at 37°C. Subsequently, the labeled target cells were washed 3 times with 5 ml RPMI and then used in the cytotoxicity assay. The assay was performed in a 96-well plate in a total volume of 200 µl supplemented RPMI with an E:T ratio of 10:1 in the presence of 10% HSA (Human-Albumin, 20%, CSL Behring GmbH: PZN-1468366). A starting concentration of 0.01 - 1 µg/ml of purified bispecific antibody and threefold dilutions thereof were used. Incubation time for the assay was 18 hours. Cytotoxicity was determined as relative values of released chromium in the supernatant relative to the difference of maximum lysis (addition of Triton-X) and spontaneous lysis (without effector cells). All measurements were carried out in quadruplicates. Measurement of chromium activity in the supernatants was performed in a Wizard 3" gamma counter (Perkin Elmer Life Sciences GmbH, Köln, Germany). Analysis of the results was carried out with Prism 5 for Windows (version 5.0, GraphPad Software Inc., San Diego, California, USA). EC50 values calculated by the analysis program from the sigmoidal dose response curves were used for comparison of cytotoxic activity (see Figure 3).

The invention is further characterized by the following items:
1. A single chain binding molecule comprising at least three binding domains, wherein
   (a) the first binding domain is capable of binding to serum albumin and is positioned at the N-terminus of the second binding domain;
   (b) said second binding domain is capable of binding to a cell surface molecule on a target cell; and
   (c) the third binding domain is capable of binding to the T cell CD3 receptor complex.
2. The binding molecule according to item 1, wherein the three domains are consecutively on one polypeptide chain in the order from the N-terminus to the C-terminus
   - the first binding domain;
   - the second binding domain; and
   - the third binding domain.
3. A binding molecule comprising at least three binding domains comprised in one polypeptide chain in the order first domain, second domain and third domain, wherein
   (a) the first binding domain is capable of binding to serum albumin and is positioned at the N-terminus of the second binding domain;
   (b) said second binding domain is capable of binding to a cell surface molecule on a target cell; and
   (c) the third binding domain is capable of binding to the T cell CD3 receptor complex; and
   wherein the yield of monomeric binding molecule isolated from the culture supernatant of host cells producing the binding molecule is at least 1.5 times higher than the yield of monomeric binding molecule isolated from the culture supernatant of host cells producing a binding molecule comprising the binding domain capable of binding to serum albumin is at the C-terminus of the molecule.
4. The binding molecule according to any of items 1 to 3, wherein at least one of the binding domains is an scFv or single domain antibody.
5. The binding molecule according to any of items 1 to 4, wherein the molecule comprises one or more further heterologous polypeptide.
6. The binding molecule according to item 5, further comprising a His-tag.
7. The binding molecule according to any of item 6, wherein the His-tag is positioned at the C-terminus of the third binding domain.
8. The binding molecule according to any of items 1 to 7, wherein
   (a) the first binding domain is capable of binding to human and non-human primate serum albumin;
   (b) the second binding domain is capable of binding to the cell surface molecule on a human and a non-human primate cell, and
   (c) the third binding domain is capable of binding to the T cell CD3 receptor complex on a human and a non-human primate cell.
9. The binding molecule according to any of items 1 to 8, wherein the first binding domain capable of binding to serum albumin is derived from a combinatorial library or an antibody binding domain.
10. The binding molecule according to any of items 1 to 9, wherein the first binding domain comprises between 10 and 25 aa residues.
11. The binding molecule according to items 1 to 10, wherein the first binding domain capable of binding to serum albumin comprises the amino acid sequence Asp-Xaa-Cys-Leu-Pro-Xaa-Trp-Gly-Cys-Leu-Trp, wherein Xaa is any amino acid.
12. The binding molecule according to items 1 to 10, wherein the first binding domain capable of binding to serum albumin is derived from a CDR of a single domain antibody.
13. The binding molecule according to any of items 1 to 12, wherein the first binding domain is binding to serum albumin with an affinity (KD) of ≤ 500 nM.
14. The binding molecule according to any of items 1 to 13, wherein
   - the second binding domain is binding to the cell surface molecule on a target cell with an affinity (KD) of ≤ 100 nM; and
   - the third binding domain is binding to the T cell CD3 receptor complex with an affinity (KD) of ≤ 100 nM.
15. The binding molecule according to any of items 1 to 14, wherein the binding molecule shows cytotoxic activity in an in vitro assay measuring the lysis of target cells by effector cells in the presence of 10% human serum albumin.
16. The binding molecule according to any of items 1 to 15, wherein the molecule consists of a single polypeptide chain.
17. The binding molecule according to any of any one of items 1 to 16, wherein
   (a) the second binding domain comprises an antibody derived VL and VH chain; and/or
   (b) the third binding domain comprises an antibody derived VL and VH chain.
18. The binding molecule according to any of items 1 to 17, wherein the first binding domain capable of binding to a cell surface molecule is binding to a tumor antigen.
19. The binding molecule according to any of items 1 to 18, wherein the second binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 of WO 2008/119567 and comprises at least the amino acid sequence Gln-Asp-Gly-Asn-Glu (SEQ ID NO:37).
20. The binding molecule according to any of items 1 to 19, characterized by an amino acid sequence as depicted in SEQ ID NOs: 8, 12, 16, 20, 24, 26, 30, or 34.
21. A method for the production of binding molecule of any of items 1 to 20, the method comprising the step of:
   - selecting for binding molecules comprising a binding domain, which is capable of binding to a cell surface molecule on a target cell, comprising at the N-terminus a binding domain which is capable of binding to serum albumin.
22. The method of item 21, further comprising the step of:
   - adding to the molecule an additional binding domain, which is capable of binding to the T cell CD3 receptor complex,.
23. A nucleic acid molecule having a sequence encoding a binding molecule as defined in any one of items 1 to 20.
24. A vector comprising a nucleic acid sequence as defined in item 23.
25. A host cell transformed or transfected with the nucleic acid sequence as defined in item 23 or with the vector as defined in item 24.
26. A process for the production of a binding molecule according to any one of items 1 to 20 or produced by a method of items 21 to 22, said process comprising culturing a host cell as defined in item 25 under conditions allowing the expression of the binding molecule as defined in any one of items 1 to 20 or produced by a method of items 21 to 22 and recovering the produced binding molecule from the culture.
27. A pharmaceutical composition comprising a binding molecule according to any one of items 1 to 20, or produced according to the process of item 22.
28. The binding molecule according to any one of items 1 to 20, or produced according to a method of items 21 to 22 for use in the prevention, treatment or amelioration of a disease selected from the group consisting of a proliferative disease, an inflammatory disease, an infectious disease and an autoimmune disease.
29. A method for the treatment or amelioration of a disease selected from the group consisting of a proliferative disease, an inflammatory disease, an infectious disease and an autoimmune disease, comprising the step of administering to a subject in need thereof the binding molecule according to any one of items 1 to 20, or produced according to a method of items 21 to 22.
30. A kit comprising a binding molecule as defined in any one of items 1 to 20 or produced according to a method of items 21 to 22, a nucleic acid molecule as defined in item 23, a vector as defined in item 24, or a host cell as defined in item 25.

**Sequence Table**

| SEQ ID NO. | DESIGNATION | SOURCE | TYPE | SEQUENCE |
|---|---|---|---|---|
| 1 | SA08 | artificial | nt | CAGGGCCTGATCGGCGACATCTGCCTGCCCAGATGGGGCTGCCTGTGG GGCGACTCCGTGAAA |
| 2 | SA08 | artificial | aa | QGLIGDICLPRWGCLWGDSVK |
| 3 | SA21 | artificial | nt | CGGCTGATCGAGGACATCTGCCTGCCCAGATGGGGCTGCCTGTGGGAG GACGAC |
| 4 | SA21 | artificial | aa | RLIEDICLPRWGCLWEDD |
| 5 | SA25 | artificial | nt | GAGGACATCTGCCTGCCCAGATGGGGCTGCCTGTGGGAGGAC |
| 6 | SA25 | artificial | aa | EDICLPRWGCLWED |
| 7 | SA21 x CD33 x I2C | artificial | nt | CGGCTGATCGAGGACATCTGCCTGCCCAGATGGGGCTGCCTGTGGGAG GACGACCAGGTGCAGCTGGTGCAGTCTGGAGCTGAGGTGAAGAAGCCT GGAGAGTCAGTCAAGGTCTCCTGCAAGGCTAGCGGGTATACCTTCACA AACTATGGAATGAACTGGGTGAAGCAGGCTCCAGGACAGGGTTTAGAG TGGATGGGCTGGATAAACACCTACACTGGAGAGCCAACCTATGCTGAT AAGTTCCAGGGACGCGTTACCATGACTACGGATACCTCTACCAGCACT GCCTATATGGAAATCCGCAACCTCGGAGGTGATGACACGGCTGTATAT TACTGTGCGCGCTGGAGTTGGAGTGATGGTTACTACGTTTACTTTGAC TACTGGGGCCAAGGCACTTCGGTCACCGTCTCCTCAGGTGGTGGTGGT TCTGGCGGCGGCGGCTCCGGTGGTGGTGGTTCTGACATCGTGATGACA CAGTCTCCAGACTCCCTGACTGTGTCTCTGGGCGAGAGGACCACCATC AACTGCAAGTCCAGCCAGAGTGTTTTAGACAGCTCCACGAATAAGAAC TCCTTAGCTTGGTACCAGCAGAAACCAGGACAGCCTCCTAAATTACTC CTTTCCTGGGCATCTACGCGGGAATCCGGGATCCCTGACCGATTCAGT GGCAGCGGGTCTGGGACAGATTTCACTCTCACTATTGACAGCCCGCAG CCTGAAGATTCTGCAACTTACTATTGTCAACAGTCTGCCCACTTCCCG ATCACCTTTGGCCAAGGGACACGACTGGAGATTAAATCCGGAGGTGGT GGATCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTACATCATCACCATCAT CAT |
| 8 | SA21 x CD33 x I2C | artificial | aa | RLIEDICLPRWGCLWEDDQVQLVQSGAEVKKPGESVKVSCKASGYTFT NYGMNWVKQAPGQGLEWMGWINTYTGEPTYADKFQGRVTMTTDTSTST AYMEIRNLGGDDTAVYYCARWSWSDGYYVYFDYWGQGTSVTVSSGGGG SGGGGSGGGGSDIVMTQSPDSLTVSLGERTTINCKSSQSVLDSSTNKN SLAWYQQKPGQPPKLLLSWASTRESGIPDRFSGSGSGTDFTLTIDSPQ PEDSATYYCQQSAHFPITFGQGTRLEIKSGGGGSEVQLVESGGGLVQP GGSLKLSCAASGFTFNKYAMNWVRQAPGKGLEWVARIRSKYNNYATYY ADSVKDRFTISRDDSKNTAYLQMNNLKTEDTAVYYCVRHGNFGNSYIS YWAYWGQGTLVTVSSGGGGSGGGGSGGGGSQTVVTQEPSLTVSPGGTV TLTCGSSTGAVTSGNYPNWVQQKPGQAPRGLIGGTKFLAPGTPARFSG SLLGGKAALTLSGVQPEDEAEYYCVLWYSNRWVFGGGTKLTVLHHHHH H |
| 9 | CD33 x I2C x | artificial | nt | CAGGTGCAGCTGGTGCAGTCTGGAGCTGAGGTGAAGAAGCCTGGAGAG |
| | SA21 | | | TCAGTCAAGGTCTCCTGCAAGGCTAGCGGGTATACCTTCACAAACTAT GGAATGAACTGGGTGAAGCAGGCTCCAGGACAGGGTTTAGAGTGGATG GGCTGGATAAACACCTACACTGGAGAGCCAACCTATGCTGATAAGTTC CAGGGACGCGTTACCATGACTACGGATACCTCTACCAGCACTGCCTAT ATGGAAATCCGCAACCTCGGAGGTGATGACACGGCTGTATATTACTGT GCGCGCTGGAGTTGGAGTGATGGTTACTACGTTTACTTTGACTACTGG GGCCAAGGCACTTCGGTCACCGTCTCCTCAGGTGGTGGTGGTTCTGGC GGCGGCGGCTCCGGTGGTGGTGGTTCTGACATCGTGATGACACAGTCT CCAGACTCCCTGACTGTGTCTCTGGGCGAGAGGACCACCATCAACTGC AAGTCCAGCCAGAGTGTTTTAGACAGCTCCACGAATAAGAACTCCTTA GCTTGGTACCAGCAGAAACCAGGACAGCCTCCTAAATTACTCCTTTCC TGGGCATCTACGCGGGAATCCGGGATCCCTGACCGATTCAGTGGCAGC GGGTCTGGGACAGATTTCACTCTCACTATTGACAGCCCGCAGCCTGAA GATTCTGCAACTTACTATTGTCAACAGTCTGCCCACTTCCCGATCACC TTTGGCCAAGGGACACGACTGGAGATTAAATCCGGAGGTGGTGGATCC GAGGTGCAGCTGGTCGAGTCTGGAGGAGGATTGGTGCAGCCTGGAGGG TCATTGAAACTCTCATGTGCAGCCTCTGGATTCACCTTCAATAAGTAC GCCATGAACTGGGTCCGCCAGGCTCCAGGAAAGGGTTTGGAATGGGTT GCTCGCATAAGAAGTAAATATAATAATTATGCAACATATTATGCCGAT TCAGTGAAAGACAGGTTCACCATCTCCAGAGATGATTCAAAAAACACT GCCTATCTACAAATGAACAACTTGAAGACTGAGGACACTGCCGTGTAC TACTGTGTGAGACATGGGAACTTCGGTAATAGCTACATATCCTACTGG GCTTACTGGGGCCAAGGGACTCTGGTCACCGTCTCCTCAGGTGGTGGT GGTTCTGGCGGCGGCGGCTCCGGTGGTGGTGGTTCTCAGACTGTTGTG ACTCAGGAACCTTCACTCACCGTATCACCTGGTGGAACAGTCACACTC ACTTGTGGCTCCTCGACTGGGGCTGTTACATCTGGCAACTACCCAAAC TGGGTCCAACAAAAACCAGGTCAGGCACCCCGTGGTCTAATAGGTGGG ACTAAGTTCCTCGCCCCCGGTACTCCTGCCAGATTCTCAGGCTCCCTG CTTGGAGGCAAGGCTGCCCTCACCCTCTCAGGGGTACAGCCAGAGGAT GAGGCAGAATATTACTGTGTTCTATGGTACAGCAACCGCTGGGTGTTC GGTGGAGGAACCAAACTGACTGTCCTACGGCTGATCGAGGACATCTGC CTGCCCAGATGGGGCTGCCTGTGGGAGGACGACCATCATCACCATCAT CAT |
| 10 | CD33 x I2C x SA21 | artificial | aa | QVQLVQSGAEVKKPGESVKVSCKASGYTFTNYGMNWVKQAPGQGLEWM GWINTYTGEPTYADKFQGRVTMTTDTSTSTAYMEIRNLGGDDTAVYYC ARWSWSDGYYVYFDYWGQGTSVTVSSGGGGSGGGGSGGGGSDIVMTQS PDSLTVSLGERTTINCKSSQSVLDSSTNKNSLAWYQQKPGQPPKLLLS WASTRESGIPDRFSGSGSGTDFTLTIDSPQPEDSATYYCQQSAHFPIT FGQGTRLEIKSGGGGSEVQLVESGGGLVQPGGSLKLSCAASGFTFNKY AMNWVRQAPGKGLEWVARIRSKYNNYATYYADSVKDRFTISRDDSKNT AYLQMNNLKTEDTAVYYCVRHGNFGNSYISYWAYWGQGTLVTVSSGGG GSGGGGSGGGGSQTVVTQEPSLTVSPGGTVTLTCGSSTGAVTSGNYPN WVQQKPGQAPRGLIGGTKFLAPGTPARFSGSLLGGKAALTLSGVQPED EAEYYCVLWYSNRWVFGGGTKLTVLRLIEDICLPRWGCLWEDDHHHHH H |
| 11 | SA21 x EpCAM x CD3 | artificial | nt | CGGCTGATCGAGGACATCTGCCTGCCCAGATGGGGCTGCCTGTGGGAG GACGACGAGCTCGTGATGACACAGTCTCCATCCTCCCTGACTGTGACA GCAGGAGAGAAGGTCACTATGAGCTGCAAGTCCAGTCAGAGTCTGTTA AACAGTGGAAATCAAAAGAACTACTTGACCTGGTACCAGCAGAAACCA GGGCAGCCTCCTAAACTGTTGATCTACTGGGCATCCACTAGGGAATCT GGGGTCCCTGATCGCTTCACAGGCAGTGGATCTGGAACAGATTTCACT CTCACCATCAGCAGTGTGCAGGCTGAAGACCTGGCAGTTTATTACTGT CAGAATGATTATAGTTATCCGCTCACGTTCGGTGCTGGGACCAAGCTT GAGATCAAAGGTGGTGGTGGTTCTGGCGGCGGCGGCTCCGGTGGTGGT GGTTCTGAGGTGCAGCTGCTCGAGCAGTCTGGAGCTGAGCTGGTAAGG CCTGGGACTTCAGTGAAGATATCCTGCAAGGCTTCTGGATACGCCTTC ACTAACTACTGGCTAGGTTGGGTAAAGCAGAGGCCTGGACATGGACTT GAGTGGATTGGAGATATTTTCCCTGGAAGTGGTAATATCCACTACAAT GAGAAGTTCAAGGGCAAAGCCACACTGACTGCAGACAAATCTTCGAGC ACAGCCTATATGCAGCTCAGTAGCCTGACATTTGAGGACTCTGCTGTC TATTTCTGTGCAAGACTGAGGAACTGGGACGAGCCTATGGACTACTGG GGCCAAGGGACCACGGTCACCGTCTCCTCCGGAGGTGGTGGCTCCGAC GTCCAACTGGTGCAGTCAGGGGCTGAAGTGAAAAAACCTGGGGCCTCA GTGAAGGTGTCCTGCAAGGCTTCTGGCTACACCTTTACTAGGTACACG |
| | | | | ATGCACTGGGTAAGGCAGGCACCTGGACAGGGTCTGGAATGGATTGGA TACATTAATCCTAGCCGTGGTTATACTAATTACGCAGACAGCGTCAAG GGCCGCTTCACAATCACTACAGACAAATCCACCAGCACAGCCTACATG GAACTGAGCAGCCTGCGTTCTGAGGACACTGCAACCTATTACTGTGCA AGATATTATGATGATCATTACTGCCTTGACTACTGGGGCCAAGGCACC ACGGTCACCGTCTCCTCAGGCGAAGGTACTAGTACTGGTTCTGGTGGA AGTGGAGGTTCAGGTGGAGCAGACGACATTGTACTGACCCAGTCTCCA GCAACTCTGTCTCTGTCTCCAGGGGAGCGTGCCACCCTGAGCTGCAGA GCCAGTCAAAGTGTAAGTTACATGAACTGGTACCAGCAGAAGCCGGGC AAGGCACCCAAAAGATGGATTTATGACACATCCAAAGTGGCTTCTGGA GTCCCTGCTCGCTTCAGTGGCAGTGGGTCTGGGACCGACTACTCTCTC ACAATCAACAGCTTGGAGGCTGAAGATGCTGCCACTTATTACTGCCAA CAGTGGAGTAGTAACCCGCTCACGTTCGGTGGCGGGACCAAGGTGGAG ATCAAACATCATCACCATCATCAT |
| 12 | SA21 x EpCAM x CD3 | artificial | aa | RLIEDICLPRWGCLWEDDELVMTQSPSSLTVTAGEKVTMSCKSSQSLL NSGNQKNYLTWYQQKPGQPPKLLIYWASTRESGVPDRFTGSGSGTDFT LTISSVQAEDLAVYYCQNDYSYPLTFGAGTKLEIKGGGGSGGGGSGGG GSEVQLLEQSGAELVRPGTSVKISCKASGYAFTNYWLGWVKQRPGHGL EWIGDIFPGSGNIHYNEKFKGKATLTADKSSSTAYMQLSSLTFEDSAV YFCARLRNWDEPMDYWGQGTTVTVSSGGGGSDVQLVQSGAEVKKPGAS VKVSCKASGYTFTRYTMHWVRQAPGQGLEWIGYINPSRGYTNYADSVK GRFTITTDKSTSTAYMELSSLRSEDTATYYCARYYDDHYCLDYWGQGT TVTVSSGEGTSTGSGGSGGSGGADDIVLTQSPATLSLSPGERATLSCR ASQSVSYMNWYQQKPGKAPKRWIYDTSKVASGVPARFSGSGSGTDYSL TINSLEAEDAATYYCQQWSSNPLTFGGGTKVEIKHHHHHH |
| 13 | EpCAM x CD3 x SA21 | artificial | nt | GAGCTCGTGATGACACAGTCTCCATCCTCCCTGACTGTGACAGCAGGA GAGAAGGTCACTATGAGCTGCAAGTCCAGTCAGAGTCTGTTAAACAGT GGAAATCAAAAGAACTACTTGACCTGGTACCAGCAGAAACCAGGGCAG CCTCCTAAACTGTTGATCTACTGGGCATCCACTAGGGAATCTGGGGTC CCTGATCGCTTCACAGGCAGTGGATCTGGAACAGATTTCACTCTCACC ATCAGCAGTGTGCAGGCTGAAGACCTGGCAGTTTATTACTGTCAGAAT GATTATAGTTATCCGCTCACGTTCGGTGCTGGGACCAAGCTTGAGATC AAAGGTGGTGGTGGTTCTGGCGGCGGCGGCTCCGGTGGTGGTGGTTCT GAGGTGCAGCTGCTCGAGCAGTCTGGAGCTGAGCTGGTAAGGCCTGGG ACTTCAGTGAAGATATCCTGCAAGGCTTCTGGATACGCCTTCACTAAC TACTGGCTAGGTTGGGTAAAGCAGAGGCCTGGACATGGACTTGAGTGG ATTGGAGATATTTTCCCTGGAAGTGGTAATATCCACTACAATGAGAAG TTCAAGGGCAAAGCCACACTGACTGCAGACAAATCTTCGAGCACAGCC TATATGCAGCTCAGTAGCCTGACATTTGAGGACTCTGCTGTCTATTTC TGTGCAAGACTGAGGAACTGGGACGAGCCTATGGACTACTGGGGCCAA GGGACCACGGTCACCGTCTCCTCCGGAGGTGGTGGCTCCGACGTCCAA CTGGTGCAGTCAGGGGCTGAAGTGAAAAAACCTGGGGCCTCAGTGAAG GTGTCCTGCAAGGCTTCTGGCTACACCTTTACTAGGTACACGATGCAC TGGGTAAGGCAGGCACCTGGACAGGGTCTGGAATGGATTGGATACATT AATCCTAGCCGTGGTTATACTAATTACGCAGACAGCGTCAAGGGCCGC TTCACAATCACTACAGACAAATCCACCAGCACAGCCTACATGGAACTG AGCAGCCTGCGTTCTGAGGACACTGCAACCTATTACTGTGCAAGATAT TATGATGATCATTACTGCCTTGACTACTGGGGCCAAGGCACCACGGTC ACCGTCTCCTCAGGCGAAGGTACTAGTACTGGTTCTGGTGGAAGTGGA GGTTCAGGTGGAGCAGACGACATTGTACTGACCCAGTCTCCAGCAACT CTGTCTCTGTCTCCAGGGGAGCGTGCCACCCTGAGCTGCAGAGCCAGT CAAAGTGTAAGTTACATGAACTGGTACCAGCAGAAGCCGGGCAAGGCA CCCAAAAGATGGATTTATGACACATCCAAAGTGGCTTCTGGAGTCCCT GCTCGCTTCAGTGGCAGTGGGTCTGGGACCGACTACTCTCTCACAATC AACAGCTTGGAGGCTGAAGATGCTGCCACTTATTACTGCCAACAGTGG AGTAGTAACCCGCTCACGTTCGGTGGCGGGACCAAGGTGGAGATCAAA CGGCTGATCGAGGACATCTGCCTGCCCAGATGGGGCTGCCTGTGGGAG GACGACCATCATCACCATCATCAT |
| 14 | EpCAM x CD3 x SA21 | artificial | aa | ELVMTQSPSSLTVTAGEKVTMSCKSSQSLLNSGNQKNYLTWYQQKPGQ PPKLLIYWASTRESGVPDRFTGSGSGTDFTLTISSVQAEDLAVYYCQN DYSYPLTFGAGTKLEIKGGGGSGGGGSGGGGSEVQLLEQSGAELVRPG TSVKISCKASGYAFTNYWLGWVKQRPGHGLEWIGDIFPGSGNIHYNEK FKGKATLTADKSSSTAYMQLSSLTFEDSAVYFCARLRNWDEPMDYWGQ GTTVTVSSGGGGSDVQLVQSGAEVKKPGASVKVSCKASGYTFTRYTMH |
| | | | | WVRQAPGQGLEWIGYINPSRGYTNYADSVKGRFTITTDKSTSTAYMEL SSLRSEDTATYYCARYYDDHYCLDYWGQGTTVTVSSGEGTSTGSGGSG GSGGADDIVLTQSPATLSLSPGERATLSCRASQSVSYMNWYQQKPGKA PKRWIYDTSKVASGVPARFSGSGSGTDYSLTINSLEAEDAATYYCQQW SSNPLTFGGGTKVEIKRLIEDICLPRWGCLWEDDHHHHHH |
| 15 | SA21 x CEA x I2C | artificial | nt | CGGCTGATCGAGGACATCTGCCTGCCCAGATGGGGCTGCCTGTGGGAG GACGACCAGGCCGTGCTGACTCAGCCGGCTTCCCTCTCTGCATCTCCT GGAGCATCAGCCAGTCTCACCTGCACCTTGCGCAGGGGCATCAATGTT GGTGCCTACAGTATATACTGGTACCAGCAGAAGCCAGGGAGTCCTCCC CAGTATCTCCTGAGGTACAAATCAGACTCAGATAAGCAGCAGGGCTCT GGAGTCTCCAGCCGCTTCTCTGCATCCAAAGATGCTTCGGCCAATGCA GGGATTTTACTCATCTCTGGGCTCCAGTCTGAGGATGAGGCTGACTAT TACTGTATGATTTGGCACAGCGGCGCTTCTGCGGTGTTCGGCGGAGGG ACCAAGTTGACCGTCCTAGGTGGTGGTGGTTCTGGCGGCGGCGGCTCC GGTGGTGGTGGTTCTGAGGTGCAGCTGGTCGAGTCTGGGGGAGGCTTG GTCCAGCCTGGGAGGTCCCTGAGACTCTCCTGTGCAGCGTCTGGATTC ACCGTCAGTAGCTACTGGATGCACTGGGTCCGCCAAGCTCCAGGGAAG GGGCTGGAATGGGTAGGTTTCATTAGAAACAAAGCTAATGGTGGGACA ACAGAATACGCCGCGTCTGTGAAAGGCAGATTCACCATCTCAAGAGAT GATTCCAAGAACACGCTGTATCTTCAAATGAACAGCCTGAGAGCCGAG GACACGGCCGTGTATTACTGTGCAAGAGATAGGGGGCTACGGTTCTAC TTTGACTACTGGGGCCAAGGGACCACGGTCACCGTCTCCTCATCCGGA GGTGGTGGCTCCGAGGTGCAGCTGGTCGAGTCTGGAGGAGGATTGGTG CAGCCTGGAGGGTCATTGAAACTCTCATGTGCAGCCTCTGGATTCACC TTCAATAAGTACGCCATGAACTGGGTCCGCCAGGCTCCAGGAAAGGGT TTGGAATGGGTTGCTCGCATAAGAAGTAAATATAATAATTATGCAACA TATTATGCCGATTCAGTGAAAGACAGGTTCACCATCTCCAGAGATGAT TCAAAAAACACTGCCTATCTACAAATGAACAACTTGAAGACTGAGGAC ACTGCCGTGTACTACTGTGTGAGACATGGGAACTTCGGTAATAGCTAC ATATCCTACTGGGCTTACTGGGGCCAAGGGACTCTGGTCACCGTCTCC TCAGGTGGTGGTGGTTCTGGCGGCGGCGGCTCCGGTGGTGGTGGTTCT CAGACTGTTGTGACTCAGGAACCTTCACTCACCGTATCACCTGGTGGA ACAGTCACACTCACTTGTGGCTCCTCGACTGGGGCTGTTACATCTGGC AACTACCCAAACTGGGTCCAACAAAAACCAGGTCAGGCACCCCGTGGT CTAATAGGTGGGACTAAGTTCCTCGCCCCCGGTACTCCTGCCAGATTC TCAGGCTCCCTGCTTGGAGGCAAGGCTGCCCTCACCCTCTCAGGGGTA CAGCCAGAGGATGAGGCAGAATATTACTGTGTTCTATGGTACAGCAAC CGCTGGGTGTTCGGTGGAGGAACCAAACTGACTGTCCTACATCATCAC CATCATCAT |
| 16 | SA21 x CEA x I2C | artificial | aa | RLIEDICLPRWGCLWEDDQAVLTQPASLSASPGASASLTCTLRRGINV GAYSIYWYQQKPGSPPQYLLRYKSDSDKQQGSGVSSRFSASKDASANA GILLISGLQSEDEADYYCMIWHSGASAVFGGGTKLTVLGGGGSGGGGS GGGGSEVQLVESGGGLVQPGRSLRLSCAASGFTVSSYWMHWVRQAPGK GLEWVGFIRNKANGGTTEYAASVKGRFTISRDDSKNTLYLQMNSLRAE DTAVYYCARDRGLRFYFDYWGQGTTVTVSSSGGGGSEVQLVESGGGLV QPGGSLKLSCAASGFTFNKYAMNWVRQAPGKGLEWVARIRSKYNNYAT YYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGG TVTLTCGSSTGAVTSGNYPNWVQQKPGQAPRGLIGGTKFLAPGTPARF SGSLLGGKAALTLSGVQPEDEAEYYCVLWYSNRWVFGGGTKLTVLHHH HHH |
| 17 | CEA x I2C x SA21 | artificial | nt | CAGGCCGTGCTGACTCAGCCGGCTTCCCTCTCTGCATCTCCTGGAGCA TCAGCCAGTCTCACCTGCACCTTGCGCAGGGGCATCAATGTTGGTGCC TACAGTATATACTGGTACCAGCAGAAGCCAGGGAGTCCTCCCCAGTAT CTCCTGAGGTACAAATCAGACTCAGATAAGCAGCAGGGCTCTGGAGTC TCCAGCCGCTTCTCTGCATCCAAAGATGCTTCGGCCAATGCAGGGATT TTACTCATCTCTGGGCTCCAGTCTGAGGATGAGGCTGACTATTACTGT ATGATTTGGCACAGCGGCGCTTCTGCGGTGTTCGGCGGAGGGACCAAG TTGACCGTCCTAGGTGGTGGTGGTTCTGGCGGCGGCGGCTCCGGTGGT GGTGGTTCTGAGGTGCAGCTGGTCGAGTCTGGGGGAGGCTTGGTCCAG CCTGGGAGGTCCCTGAGACTCTCCTGTGCAGCGTCTGGATTCACCGTC AGTAGCTACTGGATGCACTGGGTCCGCCAAGCTCCAGGGAAGGGGCTG GAATGGGTAGGTTTCATTAGAAACAAAGCTAATGGTGGGACAACAGAA TACGCCGCGTCTGTGAAAGGCAGATTCACCATCTCAAGAGATGATTCC |
| | | | | AAGAACACGCTGTATCTTCAAATGAACAGCCTGAGAGCCGAGGACACG GCCGTGTATTACTGTGCAAGAGATAGGGGGCTACGGTTCTACTTTGAC TACTGGGGCCAAGGGACCACGGTCACCGTCTCCTCATCCGGAGGTGGT GGCTCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTACGGCTGATCGAGGAC ATCTGCCTGCCCAGATGGGGCTGCCTGTGGGAGGACGACCATCATCAC CATCATCAT |
| 18 | CEA x I2C x SA21 | artificial | aa | QAVLTQPASLSASPGASASLTCTLRRGINVGAYSIYWYQQKPGSPPQY LLRYKSDSDKQQGSGVSSRFSASKDASANAGILLISGLQSEDEADYYC MIWHSGASAVFGGGTKLTVLGGGGSGGGGSGGGGSEVQLVESGGGLVQ PGRSLRLSCAASGFTVSSYWMHWVRQAPGKGLEWVGFIRNKANGGTTE YAASVKGRFTISRDDSKNTLYLQMNSLRAEDTAVYYCARDRGLRFYFD YWGQGTTVTVSSSGGGGSEVQLVESGGGLVQPGGSLKLSCAASGFTFN KYAMNWVRQAPGKGLEWVARIRSKYNNYATYYADSVKDRFTISRDDSK NTAYLQMNNLKTEDTAVYYCVRHGNFGNSYISYWAYWGQGTLVTVSSG GGGSGGGGSGGGGSQTVVTQEPSLTVSPGGTVTLTCGSSTGAVTSGNY PNWVQQKPGQAPRGLIGGTKFLAPGTPARFSGSLLGGKAALTLSGVQP EDEAEYYCVLWYSNRWVFGGGTKLTVL RLIEDICLPRWGCLWEDD HHHHHH |
| 19 | SA25 x CEA x I2C | artificial | nt | GAGGACATCTGCCTGCCCAGATGGGGCTGCCTGTGGGAGGACCAGGCC GTGCTGACTCAGCCGGCTTCCCTCTCTGCATCTCCTGGAGCATCAGCC AGTCTCACCTGCACCTTGCGCAGGGGCATCAATGTTGGTGCCTACAGT ATATACTGGTACCAGCAGAAGCCAGGGAGTCCTCCCCAGTATCTCCTG AGGTACAAATCAGACTCAGATAAGCAGCAGGGCTCTGGAGTCTCCAGC CGCTTCTCTGCATCCAAAGATGCTTCGGCCAATGCAGGGATTTTACTC ATCTCTGGGCTCCAGTCTGAGGATGAGGCTGACTATTACTGTATGATT TGGCACAGCGGCGCTTCTGCGGTGTTCGGCGGAGGGACCAAGTTGACC GTCCTAGGTGGTGGTGGTTCTGGCGGCGGCGGCTCCGGTGGTGGTGGT TCTGAGGTGCAGCTGGTCGAGTCTGGGGGAGGCTTGGTCCAGCCTGGG AGGTCCCTGAGACTCTCCTGTGCAGCGTCTGGATTCACCGTCAGTAGC TACTGGATGCACTGGGTCCGCCAAGCTCCAGGGAAGGGGCTGGAATGG GTAGGTTTCATTAGAAACAAAGCTAATGGTGGGACAACAGAATACGCC GCGTCTGTGAAAGGCAGATTCACCATCTCAAGAGATGATTCCAAGAAC ACGCTGTATCTTCAAATGAACAGCCTGAGAGCCGAGGACACGGCCGTG TATTACTGTGCAAGAGATAGGGGGCTACGGTTCTACTTTGACTACTGG GGCCAAGGGACCACGGTCACCGTCTCCTCATCCGGAGGTGGTGGCTCC GAGGTGCAGCTGGTCGAGTCTGGAGGAGGATTGGTGCAGCCTGGAGGG TCATTGAAACTCTCATGTGCAGCCTCTGGATTCACCTTCAATAAGTAC GCCATGAACTGGGTCCGCCAGGCTCCAGGAAAGGGTTTGGAATGGGTT GCTCGCATAAGAAGTAAATATAATAATTATGCAACATATTATGCCGAT TCAGTGAAAGACAGGTTCACCATCTCCAGAGATGATTCAAAAAACACT GCCTATCTACAAATGAACAACTTGAAGACTGAGGACACTGCCGTGTAC TACTGTGTGAGACATGGGAACTTCGGTAATAGCTACATATCCTACTGG GCTTACTGGGGCCAAGGGACTCTGGTCACCGTCTCCTCAGGTGGTGGT GGTTCTGGCGGCGGCGGCTCCGGTGGTGGTGGTTCTCAGACTGTTGTG ACTCAGGAACCTTCACTCACCGTATCACCTGGTGGAACAGTCACACTC ACTTGTGGCTCCTCGACTGGGGCTGTTACATCTGGCAACTACCCAAAC TGGGTCCAACAAAAACCAGGTCAGGCACCCCGTGGTCTAATAGGTGGG ACTAAGTTCCTCGCCCCCGGTACTCCTGCCAGATTCTCAGGCTCCCTG CTTGGAGGCAAGGCTGCCCTCACCCTCTCAGGGGTACAGCCAGAGGAT |
| | | | | GAGGCAGAATATTACTGTGTTCTATGGTACAGCAACCGCTGGGTGTTC GGTGGAGGAACCAAACTGACTGTCCTA CATCATCACCATCATCAT |
| 20 | SA25 x CEA x I2C | artificial | aa | EDICLPRWGCLWEDQAVLTQPASLSASPGASASLTCTLRRGINVGAYS IYWYQQKPGSPPQYLLRYKSDSDKQQGSGVSSRFSASKDASANAGILL ISGLQSEDEADYYCMIWHSGASAVFGGGTKLTVLGGGGSGGGGSGGGG SEVQLVESGGGLVQPGRSLRLSCAASGFTVSSYWMHWVRQAPGKGLEW VGFIRNKANGGTTEYAASVKGRFTISRDDSKNTLYLQMNSLRAEDTAV YYCARDRGLRFYFDYWGQGTTVTVSSSGGGGSEVQLVESGGGLVQPGG SLKLSCAASGFTFNKYAMNWVRQAPGKGLEWVARIRSKYNNYATYYAD SVKDRFTISRDDSKNTAYLQMNNLKTEDTAVYYCVRHGNFGNSYISYW AYWGQGTLVTVSSGGGGSGGGGSGGGGSQTVVTQEPSLTVSPGGTVTL TCGSSTGAVTSGNYPNWVQQKPGQAPRGLIGGTKFLAPGTPARFSGSL LGGKAALTLSGVQPEDEAEYYCVLWYSNRWVFGGGTKLTVLHHHHHH |
| 21 | CEA x I2C x SA25 | artificial | nt | CAGGCCGTGCTGACTCAGCCGGCTTCCCTCTCTGCATCTCCTGGAGCA TCAGCCAGTCTCACCTGCACCTTGCGCAGGGGCATCAATGTTGGTGCC TACAGTATATACTGGTACCAGCAGAAGCCAGGGAGTCCTCCCCAGTAT CTCCTGAGGTACAAATCAGACTCAGATAAGCAGCAGGGCTCTGGAGTC TCCAGCCGCTTCTCTGCATCCAAAGATGCTTCGGCCAATGCAGGGATT TTACTCATCTCTGGGCTCCAGTCTGAGGATGAGGCTGACTATTACTGT ATGATTTGGCACAGCGGCGCTTCTGCGGTGTTCGGCGGAGGGACCAAG TTGACCGTCCTAGGTGGTGGTGGTTCTGGCGGCGGCGGCTCCGGTGGT GGTGGTTCTGAGGTGCAGCTGGTCGAGTCTGGGGGAGGCTTGGTCCAG CCTGGGAGGTCCCTGAGACTCTCCTGTGCAGCGTCTGGATTCACCGTC AGTAGCTACTGGATGCACTGGGTCCGCCAAGCTCCAGGGAAGGGGCTG GAATGGGTAGGTTTCATTAGAAACAAAGCTAATGGTGGGACAACAGAA TACGCCGCGTCTGTGAAAGGCAGATTCACCATCTCAAGAGATGATTCC AAGAACACGCTGTATCTTCAAATGAACAGCCTGAGAGCCGAGGACACG GCCGTGTATTACTGTGCAAGAGATAGGGGGCTACGGTTCTACTTTGAC TACTGGGGCCAAGGGACCACGGTCACCGTCTCCTCATCCGGAGGTGGT GGCTCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTAGAGGACATCTGCCTG CCCAGATGGGGCTGCCTGTGGGAGGAC CATCATCACCATCATCAT |
| 22 | CEA x I2C x SA25 | artificial | aa | QAVLTQPASLSASPGASASLTCTLRRGINVGAYSIYWYQQKPGSPPQY LLRYKSDSDKQQGSGVSSRFSASKDASANAGILLISGLQSEDEADYYC MIWHSGASAVFGGGTKLTVLGGGGSGGGGSGGGGSEVQLVESGGGLVQ PGRSLRLSCAASGFTVSSYWMHWVRQAPGKGLEWVGFIRNKANGGTTE YAASVKGRFTISRDDSKNTLYLQMNSLRAEDTAVYYCARDRGLRFYFD YWGQGTTVTVSSSGGGGSEVQLVESGGGLVQPGGSLKLSCAASGFTFN KYAMNWVRQAPGKGLEWVARIRSKYNNYATYYADSVKDRFTISRDDSK NTAYLQMNNLKTEDTAVYYCVRHGNFGNSYISYWAYWGQGTLVTVSSG GGGSGGGGSGGGGSQTVVTQEPSLTVSPGGTVTLTCGSSTGAVTSGNY PNWVQQKPGQAPRGLIGGTKFLAPGTPARFSGSLLGGKAALTLSGVQP EDEAEYYCVLWYSNRWVFGGGTKLTVLEDICLPRWGCLWEDHHHHHH |
| 23 | SA08 x CEA x I2C | artificial | nt | CAGGGCCTGATCGGCGACATCTGCCTGCCCAGATGGGGCTGCCTGTGG GGCGACTCCGTGAAACAGGCCGTGCTGACTCAGCCGGCTTCCCTCTCT GCATCTCCTGGAGCATCAGCCAGTCTCACCTGCACCTTGCGCAGGGGC ATCAATGTTGGTGCCTACAGTATATACTGGTACCAGCAGAAGCCAGGG AGTCCTCCCCAGTATCTCCTGAGGTACAAATCAGACTCAGATAAGCAG CAGGGCTCTGGAGTCTCCAGCCGCTTCTCTGCATCCAAAGATGCTTCG GCCAATGCAGGGATTTTACTCATCTCTGGGCTCCAGTCTGAGGATGAG |
| | | | | GCTGACTATTACTGTATGATTTGGCACAGCGGCGCTTCTGCGGTGTTC GGCGGAGGGACCAAGTTGACCGTCCTAGGTGGTGGTGGTTCTGGCGGC GGCGGCTCCGGTGGTGGTGGTTCTGAGGTGCAGCTGGTCGAGTCTGGG GGAGGCTTGGTCCAGCCTGGGAGGTCCCTGAGACTCTCCTGTGCAGCG TCTGGATTCACCGTCAGTAGCTACTGGATGCACTGGGTCCGCCAAGCT CCAGGGAAGGGGCTGGAATGGGTAGGTTTCATTAGAAACAAAGCTAAT GGTGGGACAACAGAATACGCCGCGTCTGTGAAAGGCAGATTCACCATC TCAAGAGATGATTCCAAGAACACGCTGTATCTTCAAATGAACAGCCTG AGAGCCGAGGACACGGCCGTGTATTACTGTGCAAGAGATAGGGGGCTA CGGTTCTACTTTGACTACTGGGGCCAAGGGACCACGGTCACCGTCTCC TCATCCGGAGGTGGTGGCTCCGAGGTGCAGCTGGTCGAGTCTGGAGGA GGATTGGTGCAGCCTGGAGGGTCATTGAAACTCTCATGTGCAGCCTCT GGATTCACCTTCAATAAGTACGCCATGAACTGGGTCCGCCAGGCTCCA GGAAAGGGTTTGGAATGGGTTGCTCGCATAAGAAGTAAATATAATAAT TATGCAACATATTATGCCGATTCAGTGAAAGACAGGTTCACCATCTCC AGAGATGATTCAAAAAACACTGCCTATCTACAAATGAACAACTTGAAG ACTGAGGACACTGCCGTGTACTACTGTGTGAGACATGGGAACTTCGGT AATAGCTACATATCCTACTGGGCTTACTGGGGCCAAGGGACTCTGGTC ACCGTCTCCTCAGGTGGTGGTGGTTCTGGCGGCGGCGGCTCCGGTGGT GGTGGTTCTCAGACTGTTGTGACTCAGGAACCTTCACTCACCGTATCA CCTGGTGGAACAGTCACACTCACTTGTGGCTCCTCGACTGGGGCTGTT ACATCTGGCAACTACCCAAACTGGGTCCAACAAAAACCAGGTCAGGCA CCCCGTGGTCTAATAGGTGGGACTAAGTTCCTCGCCCCCGGTACTCCT GCCAGATTCTCAGGCTCCCTGCTTGGAGGCAAGGCTGCCCTCACCCTC TCAGGGGTACAGCCAGAGGATGAGGCAGAATATTACTGTGTTCTATGG TACAGCAACCGCTGGGTGTTCGGTGGAGGAACCAAACTGACTGTCCTA CATCATCACCATCATCAT |
| 24 | SA08 x CEA x I2C | artificial | aa | QGLIGDICLPRWGCLWGDSVKQAVLTQPASLSASPGASASLTCTLRRG INVGAYSIYWYQQKPGSPPQYLLRYKSDSDKQQGSGVSSRFSASKDAS ANAGILLISGLQSEDEADYYCMIWHSGASAVFGGGTKLTVLGGGGSGG GGSGGGGSEVQLVESGGGLVQPGRSLRLSCAASGFTVSSYWMHWVRQA PGKGLEWVGFIRNKANGGTTEYAASVKGRFTISRDDSKNTLYLQMNSL RAEDTAVYYCARDRGLRFYFDYWGQGTTVTVSSSGGGGSEVQLVESGG GLVQPGGSLKLSCAASGFTFNKYAMNWVRQAPGKGLEWVARIRSKYNN YATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVS PGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL HHHHHH |
| 25 | SA21 x CEA x I2C H0 | artificial | nt | CGGCTGATCGAGGACATCTGCCTGCCCAGATGGGGCTGCCTGTGGGAG GACGACCAGGCCGTGCTGACTCAGCCGGCTTCCCTCTCTGCATCTCCT GGAGCATCAGCCAGTCTCACCTGCACCTTGCGCAGGGGCATCAATGTT GGTGCCTACAGTATATACTGGTACCAGCAGAAGCCAGGGAGTCCTCCC CAGTATCTCCTGAGGTACAAATCAGACTCAGATAAGCAGCAGGGCTCT GGAGTCTCCAGCCGCTTCTCTGCATCCAAAGATGCTTCGGCCAATGCA GGGATTTTACTCATCTCTGGGCTCCAGTCTGAGGATGAGGCTGACTAT TACTGTATGATTTGGCACAGCGGCGCTTCTGCGGTGTTCGGCGGAGGG ACCAAGTTGACCGTCCTAGGTGGTGGTGGTTCTGGCGGCGGCGGCTCC GGTGGTGGTGGTTCTGAGGTGCAGCTGGTCGAGTCTGGGGGAGGCTTG GTCCAGCCTGGGAGGTCCCTGAGACTCTCCTGTGCAGCGTCTGGATTC ACCGTCAGTAGCTACTGGATGCACTGGGTCCGCCAAGCTCCAGGGAAG GGGCTGGAATGGGTAGGTTTCATTAGAAACAAAGCTAATGGTGGGACA ACAGAATACGCCGCGTCTGTGAAAGGCAGATTCACCATCTCAAGAGAT GATTCCAAGAACACGCTGTATCTTCAAATGAACAGCCTGAGAGCCGAG GACACGGCCGTGTATTACTGTGCAAGAGATAGGGGGCTACGGTTCTAC TTTGACTACTGGGGCCAAGGGACCACGGTCACCGTCTCCTCATCCGGA GGTGGTGGCTCCGAGGTGCAGCTGGTCGAGTCTGGAGGAGGATTGGTG CAGCCTGGAGGGTCATTGAAACTCTCATGTGCAGCCTCTGGATTCACC TTCAATAAGTACGCCATGAACTGGGTCCGCCAGGCTCCAGGAAAGGGT TTGGAATGGGTTGCTCGCATAAGAAGTAAATATAATAATTATGCAACA TATTATGCCGATTCAGTGAAAGACAGGTTCACCATCTCCAGAGATGAT TCAAAAAACACTGCCTATCTACAAATGAACAACTTGAAGACTGAGGAC ACTGCCGTGTACTACTGTGTGAGACATGGGAACTTCGGTAATAGCTAC ATATCCTACTGGGCTTACTGGGGCCAAGGGACTCTGGTCACCGTCTCC |
| | | | | TCAGGTGGTGGTGGTTCTGGCGGCGGCGGCTCCGGTGGTGGTGGTTCT CAGACTGTTGTGACTCAGGAACCTTCACTCACCGTATCACCTGGTGGA ACAGTCACACTCACTTGTGGCTCCTCGACTGGGGCTGTTACATCTGGC AACTACCCAAACTGGGTCCAACAAAAACCAGGTCAGGCACCCCGTGGT CTAATAGGTGGGACTAAGTTCCTCGCCCCCGGTACTCCTGCCAGATTC TCAGGCTCCCTGCTTGGAGGCAAGGCTGCCCTCACCCTCTCAGGGGTA CAGCCAGAGGATGAGGCAGAATATTACTGTGTTCTATGGTACAGCAAC CGCTGGGTGTTCGGTGGAGGAACCAAACTGACTGTCCTA |
| 26 | SA21 x CEA x I2C H0 | artificial | aa | RLIEDICLPRWGCLWEDDQAVLTQPASLSASPGASASLTCTLRRGINV GAYSIYWYQQKPGSPPQYLLRYKSDSDKQQGSGVSSRFSASKDASANA GILLISGLQSEDEADYYCMIWHSGASAVFGGGTKLTVLGGGGSGGGGS GGGGSEVQLVESGGGLVQPGRSLRLSCAASGFTVSSYWMHWVRQAPGK GLEWVGFIRNKANGGTTEYAASVKGRFTISRDDSKNTLYLQMNSLRAE DTAVYYCARDRGLRFYFDYWGQGTTVTVSSSGGGGSEVQLVESGGGLV QPGGSLKLSCAASGFTFNKYAMNWVRQAPGKGLEWVARIRSKYNNYAT YYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGG TVTLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 27 | CEA x I2C x SA21 H0 | artificial | nt | CAGGCCGTGCTGACTCAGCCGGCTTCCCTCTCTGCATCTCCTGGAGCA TCAGCCAGTCTCACCTGCACCTTGCGCAGGGGCATCAATGTTGGTGCC TACAGTATATACTGGTACCAGCAGAAGCCAGGGAGTCCTCCCCAGTAT CTCCTGAGGTACAAATCAGACTCAGATAAGCAGCAGGGCTCTGGAGTC TCCAGCCGCTTCTCTGCATCCAAAGATGCTTCGGCCAATGCAGGGATT TTACTCATCTCTGGGCTCCAGTCTGAGGATGAGGCTGACTATTACTGT ATGATTTGGCACAGCGGCGCTTCTGCGGTGTTCGGCGGAGGGACCAAG TTGACCGTCCTAGGTGGTGGTGGTTCTGGCGGCGGCGGCTCCGGTGGT GGTGGTTCTGAGGTGCAGCTGGTCGAGTCTGGGGGAGGCTTGGTCCAG CCTGGGAGGTCCCTGAGACTCTCCTGTGCAGCGTCTGGATTCACCGTC AGTAGCTACTGGATGCACTGGGTCCGCCAAGCTCCAGGGAAGGGGCTG GAATGGGTAGGTTTCATTAGAAACAAAGCTAATGGTGGGACAACAGAA TACGCCGCGTCTGTGAAAGGCAGATTCACCATCTCAAGAGATGATTCC AAGAACACGCTGTATCTTCAAATGAACAGCCTGAGAGCCGAGGACACG GCCGTGTATTACTGTGCAAGAGATAGGGGGCTACGGTTCTACTTTGAC TACTGGGGCCAAGGGACCACGGTCACCGTCTCCTCATCCGGAGGTGGT GGCTCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTACGGCTGATCGAGGAC ATCTGCCTGCCCAGATGGGGCTGCCTGTGGGAGGACGAC |
| 28 | CEA x I2C x SA21 H0 | artificial | aa | QAVLTQPASLSASPGASASLTCTLRRGINVGAYSIYWYQQKPGSPPQY LLRYKSDSDKQQGSGVSSRFSASKDASANAGILLISGLQSEDEADYYC MIWHSGASAVFGGGTKLTVLGGGGSGGGGSGGGGSEVQLVESGGGLVQ PGRSLRLSCAASGFTVSSYWMHWVRQAPGKGLEWVGFIRNKANGGTTE YAASVKGRFTISRDDSKNTLYLQMNSLRAEDTAVYYCARDRGLRFYFD YWGQGTTVTVSSSGGGGSEVQLVESGGGLVQPGGSLKLSCAASGFTFN KYAMNWVRQAPGKGLEWVARIRSKYNNYATYYADSVKDRFTISRDDSK NTAYLQMNNLKTEDTAVYYCVRHGNFGNSYISYWAYWGQGTLVTVSSG GGGSGGGGSGGGGSQTVVTQEPSLTVSPGGTVTLTCGSSTGAVTSGNY PNWVQQKPGQAPRGLIGGTKFLAPGTPARFSGSLLGGKAALTLSGVQP EDEAEYYCVLWYSNRWVFGGGTKLTVL RLIEDICLPRWGCLWEDD |
| 29 | SA25 x CEA x | artificial | nt | GAGGACATCTGCCTGCCCAGATGGGGCTGCCTGTGGGAGGACCAGGCC |
| | I2C H0 | | | GTGCTGACTCAGCCGGCTTCCCTCTCTGCATCTCCTGGAGCATCAGCC AGTCTCACCTGCACCTTGCGCAGGGGCATCAATGTTGGTGCCTACAGT ATATACTGGTACCAGCAGAAGCCAGGGAGTCCTCCCCAGTATCTCCTG AGGTACAAATCAGACTCAGATAAGCAGCAGGGCTCTGGAGTCTCCAGC CGCTTCTCTGCATCCAAAGATGCTTCGGCCAATGCAGGGATTTTACTC ATCTCTGGGCTCCAGTCTGAGGATGAGGCTGACTATTACTGTATGATT TGGCACAGCGGCGCTTCTGCGGTGTTCGGCGGAGGGACCAAGTTGACC GTCCTAGGTGGTGGTGGTTCTGGCGGCGGCGGCTCCGGTGGTGGTGGT TCTGAGGTGCAGCTGGTCGAGTCTGGGGGAGGCTTGGTCCAGCCTGGG AGGTCCCTGAGACTCTCCTGTGCAGCGTCTGGATTCACCGTCAGTAGC TACTGGATGCACTGGGTCCGCCAAGCTCCAGGGAAGGGGCTGGAATGG GTAGGTTTCATTAGAAACAAAGCTAATGGTGGGACAACAGAATACGCC GCGTCTGTGAAAGGCAGATTCACCATCTCAAGAGATGATTCCAAGAAC ACGCTGTATCTTCAAATGAACAGCCTGAGAGCCGAGGACACGGCCGTG TATTACTGTGCAAGAGATAGGGGGCTACGGTTCTACTTTGACTACTGG GGCCAAGGGACCACGGTCACCGTCTCCTCATCCGGAGGTGGTGGCTCC GAGGTGCAGCTGGTCGAGTCTGGAGGAGGATTGGTGCAGCCTGGAGGG TCATTGAAACTCTCATGTGCAGCCTCTGGATTCACCTTCAATAAGTAC GCCATGAACTGGGTCCGCCAGGCTCCAGGAAAGGGTTTGGAATGGGTT GCTCGCATAAGAAGTAAATATAATAATTATGCAACATATTATGCCGAT TCAGTGAAAGACAGGTTCACCATCTCCAGAGATGATTCAAAAAACACT GCCTATCTACAAATGAACAACTTGAAGACTGAGGACACTGCCGTGTAC TACTGTGTGAGACATGGGAACTTCGGTAATAGCTACATATCCTACTGG GCTTACTGGGGCCAAGGGACTCTGGTCACCGTCTCCTCAGGTGGTGGT GGTTCTGGCGGCGGCGGCTCCGGTGGTGGTGGTTCTCAGACTGTTGTG ACTCAGGAACCTTCACTCACCGTATCACCTGGTGGAACAGTCACACTC ACTTGTGGCTCCTCGACTGGGGCTGTTACATCTGGCAACTACCCAAAC TGGGTCCAACAAAAACCAGGTCAGGCACCCCGTGGTCTAATAGGTGGG ACTAAGTTCCTCGCCCCCGGTACTCCTGCCAGATTCTCAGGCTCCCTG CTTGGAGGCAAGGCTGCCCTCACCCTCTCAGGGGTACAGCCAGAGGAT GAGGCAGAATATTACTGTGTTCTATGGTACAGCAACCGCTGGGTGTTC GGTGGAGGAACCAAACTGACTGTCCTA |
| 30 | SA25 x CEA x I2C H0 | artificial | aa | EDICLPRWGCLWEDQAVLTQPASLSASPGASASLTCTLRRGINVGAYS IYWYQQKPGSPPQYLLRYKSDSDKQQGSGVSSRFSASKDASANAGILL ISGLQSEDEADYYCMIWHSGASAVFGGGTKLTVLGGGGSGGGGSGGGG SEVQLVESGGGLVQPGRSLRLSCAASGFTVSSYWMHWVRQAPGKGLEW VGFIRNKANGGTTEYAASVKGRFTISRDDSKNTLYLQMNSLRAEDTAV YYCARDRGLRFYFDYWGQGTTVTVSSSGGGGSEVQLVESGGGLVQPGG SLKLSCAASGFTFNKYAMNWVRQAPGKGLEWVARIRSKYNNYATYYAD SVKDRFTISRDDSKNTAYLQMNNLKTEDTAVYYCVRHGNFGNSYISYW AYWGQGTLVTVSSGGGGSGGGGSGGGGSQTVVTQEPSLTVSPGGTVTL TCGSSTGAVTSGNYPNWVQQKPGQAPRGLIGGTKFLAPGTPARFSGSL LGGKAALTLSGVQPEDEAEYYCVLWYSNRWVFGGGTKLTVL |
| 31 | CEA x I2C x SA25 H0 | artificial | nt | CAGGCCGTGCTGACTCAGCCGGCTTCCCTCTCTGCATCTCCTGGAGCA TCAGCCAGTCTCACCTGCACCTTGCGCAGGGGCATCAATGTTGGTGCC TACAGTATATACTGGTACCAGCAGAAGCCAGGGAGTCCTCCCCAGTAT CTCCTGAGGTACAAATCAGACTCAGATAAGCAGCAGGGCTCTGGAGTC TCCAGCCGCTTCTCTGCATCCAAAGATGCTTCGGCCAATGCAGGGATT TTACTCATCTCTGGGCTCCAGTCTGAGGATGAGGCTGACTATTACTGT ATGATTTGGCACAGCGGCGCTTCTGCGGTGTTCGGCGGAGGGACCAAG TTGACCGTCCTAGGTGGTGGTGGTTCTGGCGGCGGCGGCTCCGGTGGT GGTGGTTCTGAGGTGCAGCTGGTCGAGTCTGGGGGAGGCTTGGTCCAG CCTGGGAGGTCCCTGAGACTCTCCTGTGCAGCGTCTGGATTCACCGTC AGTAGCTACTGGATGCACTGGGTCCGCCAAGCTCCAGGGAAGGGGCTG GAATGGGTAGGTTTCATTAGAAACAAAGCTAATGGTGGGACAACAGAA TACGCCGCGTCTGTGAAAGGCAGATTCACCATCTCAAGAGATGATTCC AAGAACACGCTGTATCTTCAAATGAACAGCCTGAGAGCCGAGGACACG GCCGTGTATTACTGTGCAAGAGATAGGGGGCTACGGTTCTACTTTGAC TACTGGGGCCAAGGGACCACGGTCACCGTCTCCTCATCCGGAGGTGGT GGCTCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC |
| | | | | GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTAGAGGACATCTGCCTG CCCAGATGGGGCTGCCTGTGGGAGGAC |
| 32 | CEA × I2C × SA25 H0 | artificial | aa | QAVLTQPASLSASPGASASLTCTLRRGINVGAYSIYWYQQKPGSPPQY LLRYKSDSDKQQGSGVSSRFSASKDASANAGILLISGLQSEDEADYYC MIWHSGASAVFGGGTKLTVLGGGGSGGGGSGGGGSEVQLVESGGGLVQ PGRSLRLSCAASGFTVSSYWMHWVRQAPGKGLEWVGFIRNKANGGTTE YAASVKGRFTISRDDSKNTLYLQMNSLRAEDTAVYYCARDRGLRFYFD YWGQGTTVTVSSSGGGGSEVQLVESGGGLVQPGGSLKLSCAASGFTFN KYAMNWVRQAPGKGLEWVARIRSKYNNYATYYADSVKDRFTISRDDSK NTAYLQMNNLKTEDTAVYYCVRHGNFGNSYISYWAYWGQGTLVTVSSG GGGSGGGGSGGGGSQTVVTQEPSLTVSPGGTVTLTCGSSTGAVTSGNY PNWVQQKPGQAPRGLIGGTKFLAPGTPARFSGSLLGGKAALTLSGVQP EDEAEYYCVLWYSNRWVFGGGTKLTVLEDICLPRWGCLWED |
| 33 | SA08 x CEA x I2C H0 | artificial | nt | CAGGGCCTGATCGGCGACATCTGCCTGCCCAGATGGGGCTGCCTGTGG GGCGACTCCGTGAAACAGGCCGTGCTGACTCAGCCGGCTTCCCTCTCT GCATCTCCTGGAGCATCAGCCAGTCTCACCTGCACCTTGCGCAGGGGC ATCAATGTTGGTGCCTACAGTATATACTGGTACCAGCAGAAGCCAGGG AGTCCTCCCCAGTATCTCCTGAGGTACAAATCAGACTCAGATAAGCAG CAGGGCTCTGGAGTCTCCAGCCGCTTCTCTGCATCCAAAGATGCTTCG GCCAATGCAGGGATTTTACTCATCTCTGGGCTCCAGTCTGAGGATGAG GCTGACTATTACTGTATGATTTGGCACAGCGGCGCTTCTGCGGTGTTC GGCGGAGGGACCAAGTTGACCGTCCTAGGTGGTGGTGGTTCTGGCGGC GGCGGCTCCGGTGGTGGTGGTTCTGAGGTGCAGCTGGTCGAGTCTGGG GGAGGCTTGGTCCAGCCTGGGAGGTCCCTGAGACTCTCCTGTGCAGCG TCTGGATTCACCGTCAGTAGCTACTGGATGCACTGGGTCCGCCAAGCT CCAGGGAAGGGGCTGGAATGGGTAGGTTTCATTAGAAACAAAGCTAAT GGTGGGACAACAGAATACGCCGCGTCTGTGAAAGGCAGATTCACCATC TCAAGAGATGATTCCAAGAACACGCTGTATCTTCAAATGAACAGCCTG AGAGCCGAGGACACGGCCGTGTATTACTGTGCAAGAGATAGGGGGCTA CGGTTCTACTTTGACTACTGGGGCCAAGGGACCACGGTCACCGTCTCC TCATCCGGAGGTGGTGGCTCCGAGGTGCAGCTGGTCGAGTCTGGAGGA GGATTGGTGCAGCCTGGAGGGTCATTGAAACTCTCATGTGCAGCCTCT GGATTCACCTTCAATAAGTACGCCATGAACTGGGTCCGCCAGGCTCCA GGAAAGGGTTTGGAATGGGTTGCTCGCATAAGAAGTAAATATAATAAT TATGCAACATATTATGCCGATTCAGTGAAAGACAGGTTCACCATCTCC AGAGATGATTCAAAAAACACTGCCTATCTACAAATGAACAACTTGAAG ACTGAGGACACTGCCGTGTACTACTGTGTGAGACATGGGAACTTCGGT AATAGCTACATATCCTACTGGGCTTACTGGGGCCAAGGGACTCTGGTC ACCGTCTCCTCAGGTGGTGGTGGTTCTGGCGGCGGCGGCTCCGGTGGT GGTGGTTCTCAGACTGTTGTGACTCAGGAACCTTCACTCACCGTATCA CCTGGTGGAACAGTCACACTCACTTGTGGCTCCTCGACTGGGGCTGTT ACATCTGGCAACTACCCAAACTGGGTCCAACAAAAACCAGGTCAGGCA CCCCGTGGTCTAATAGGTGGGACTAAGTTCCTCGCCCCCGGTACTCCT GCCAGATTCTCAGGCTCCCTGCTTGGAGGCAAGGCTGCCCTCACCCTC TCAGGGGTACAGCCAGAGGATGAGGCAGAATATTACTGTGTTCTATGG TACAGCAACCGCTGGGTGTTCGGTGGAGGAACCAAACTGACTGTCCTA |
| 34 | SA08 x CEA x I2C H0 | artificial | aa | QGLIGDICLPRWGCLWGDSVKQAVLTQPASLSASPGASASLTCTLRRG INVGAYSIYWYQQKPGSPPQYLLRYKSDSDKQQGSGVSSRFSASKDAS ANAGILLISGLQSEDEADYYCMIWHSGASAVFGGGTKLTVLGGGGSGG GGSGGGGSEVQLVESGGGLVQPGRSLRLSCAASGFTVSSYWMHWVRQA PGKGLEWVGFIRNKANGGTTEYAASVKGRFTISRDDSKNTLYLQMNSL RAEDTAVYYCARDRGLRFYFDYWGQGTTVTVSSSGGGGSEVQLVESGG GLVQPGGSLKLSCAASGFTFNKYAMNWVRQAPGKGLEWVARIRSKYNN YATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVS |
| | | | | PGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL |
| 35 | CEA x I2C x SA08 H0 | artificial | nt | CAGGCCGTGCTGACTCAGCCGGCTTCCCTCTCTGCATCTCCTGGAGCA TCAGCCAGTCTCACCTGCACCTTGCGCAGGGGCATCAATGTTGGTGCC TACAGTATATACTGGTACCAGCAGAAGCCAGGGAGTCCTCCCCAGTAT CTCCTGAGGTACAAATCAGACTCAGATAAGCAGCAGGGCTCTGGAGTC TCCAGCCGCTTCTCTGCATCCAAAGATGCTTCGGCCAATGCAGGGATT TTACTCATCTCTGGGCTCCAGTCTGAGGATGAGGCTGACTATTACTGT ATGATTTGGCACAGCGGCGCTTCTGCGGTGTTCGGCGGAGGGACCAAG TTGACCGTCCTAGGTGGTGGTGGTTCTGGCGGCGGCGGCTCCGGTGGT GGTGGTTCTGAGGTGCAGCTGGTCGAGTCTGGGGGAGGCTTGGTCCAG CCTGGGAGGTCCCTGAGACTCTCCTGTGCAGCGTCTGGATTCACCGTC AGTAGCTACTGGATGCACTGGGTCCGCCAAGCTCCAGGGAAGGGGCTG GAATGGGTAGGTTTCATTAGAAACAAAGCTAATGGTGGGACAACAGAA TACGCCGCGTCTGTGAAAGGCAGATTCACCATCTCAAGAGATGATTCC AAGAACACGCTGTATCTTCAAATGAACAGCCTGAGAGCCGAGGACACG GCCGTGTATTACTGTGCAAGAGATAGGGGGCTACGGTTCTACTTTGAC TACTGGGGCCAAGGGACCACGGTCACCGTCTCCTCATCCGGAGGTGGT GGCTCCGAGGTGCAGCTGGTCGAGTCTGGAGGAGGATTGGTGCAGCCT GGAGGGTCATTGAAACTCTCATGTGCAGCCTCTGGATTCACCTTCAAT AAGTACGCCATGAACTGGGTCCGCCAGGCTCCAGGAAAGGGTTTGGAA TGGGTTGCTCGCATAAGAAGTAAATATAATAATTATGCAACATATTAT GCCGATTCAGTGAAAGACAGGTTCACCATCTCCAGAGATGATTCAAAA AACACTGCCTATCTACAAATGAACAACTTGAAGACTGAGGACACTGCC GTGTACTACTGTGTGAGACATGGGAACTTCGGTAATAGCTACATATCC TACTGGGCTTACTGGGGCCAAGGGACTCTGGTCACCGTCTCCTCAGGT GGTGGTGGTTCTGGCGGCGGCGGCTCCGGTGGTGGTGGTTCTCAGACT GTTGTGACTCAGGAACCTTCACTCACCGTATCACCTGGTGGAACAGTC ACACTCACTTGTGGCTCCTCGACTGGGGCTGTTACATCTGGCAACTAC CCAAACTGGGTCCAACAAAAACCAGGTCAGGCACCCCGTGGTCTAATA GGTGGGACTAAGTTCCTCGCCCCCGGTACTCCTGCCAGATTCTCAGGC TCCCTGCTTGGAGGCAAGGCTGCCCTCACCCTCTCAGGGGTACAGCCA GAGGATGAGGCAGAATATTACTGTGTTCTATGGTACAGCAACCGCTGG GTGTTCGGTGGAGGAACCAAACTGACTGTCCTACAGGGCCTGATCGGC GACATCTGCCTGCCCAGATGGGGCTGCCTGTGGGGCGACTCCGTGAAA |
| 36 | CEA x I2C x SA08 H0 | artificial | aa | QGLIGDICLPRWGCLWGDSVKQAVLTQPASLSASPGASASLTCTLRRG INVGAYSIYWYQQKPGSPPQYLLRYKSDSDKQQGSGVSSRFSASKDAS ANAGILLISGLQSEDEADYYCMIWHSGASAVFGGGTKLTVLGGGGSGG GGSGGGGSEVQLVESGGGLVQPGRSLRLSCAASGFTVSSYWMHWVRQA PGKGLEWVGFIRNKANGGTTEYAASVKGRFTISRDDSKNTLYLQMNSL RAEDTAVYYCARDRGLRFYFDYWGQGTTVTVSSSGGGGSEVQLVESGG GLVQPGGSLKLSCAASGFTFNKYAMNWVRQAPGKGLEWVARIRSKYNN YATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVS PGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL QGLIGDICLPRWGCLWGDSVK |
| 37 | N-terminus of CD3ε | human | aa | QDGNE |

## Claims

1. A single chain binding molecule comprising at least three binding domains, wherein
(a) the first binding domain is capable of binding to serum albumin and is positioned at the N-terminus of the second binding domain;
(b) said second binding domain is capable of binding to a cell surface molecule on a target cell; and
(c) the third binding domain is capable of binding to the T cell CD3 receptor complex, wherein at least one of the binding domains is an scFv or single domain antibody.

2. The binding molecule according to claim 1, wherein the three domains are consecutively on one polypeptide chain in the order from the N-terminus to the C-terminus
• the first binding domain;
• the second binding domain; and
• the third binding domain.

3. A binding molecule comprising at least three binding domains comprised in one polypeptide chain in the order first domain, second domain and third domain, wherein
(a) the first binding domain is capable of binding to serum albumin and is positioned at the N-terminus of the second binding domain;
(b) said second binding domain is capable of binding to a cell surface molecule on a target cell; and
(c) the third binding domain is capable of binding to the T cell CD3 receptor complex; and
wherein the yield of monomeric binding molecule isolated from the culture supernatant of host cells producing the binding molecule is at least 1.5 times higher than the yield of monomeric binding molecule isolated from the culture supernatant of host cells producing a binding molecule comprising the binding domain capable of binding to serum albumin is at the C-terminus of the molecule.

4. The binding molecule according to any of claims 1 to 3, wherein the molecule comprises one or more further heterologous polypeptide.

5. The binding molecule according to any of claims 1 to 4, wherein the first binding domain capable of binding to serum albumin is derived from a combinatorial library or an antibody binding domain, wherein preferably the first binding domain comprises between 10 and 25 aa residues, and/or wherein the first binding domain is preferably capable of binding to serum albumin comprises the amino acid sequence Asp-Xaa-Cys-Leu-Pro-Xaa-Trp-Gly-Cys-Leu-Trp (SEQ ID NO: 39), wherein Xaa is any amino acid, and/or wherein the first binding domain is preferably capable of binding to serum albumin is derived from a CDR of a single domain antibody, and/or wherein the first binding domain is preferably binding to serum albumin with an affinity (KD) of ≤ 500 nM.

6. The binding molecule according to any of any one of claims 1 to 5, wherein
(a) the second binding domain comprises an antibody derived VL and VH chain; and/or
(b) the third binding domain comprises an antibody derived VL and VH chain.

7. The binding molecule according to any of claims 1 to 6, wherein the first binding domain capable of binding to a cell surface molecule is binding to a tumor antigen.

8. The binding molecule according to any of claims 1 to 7, wherein the second binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 of WO 2008/119567 and comprises at least the amino acid sequence Gln-Asp-Gly-Asn-Glu (SEQ ID NO: 37), wherein the binding molecule is preferably **characterized by** an amino acid sequence as depicted in SEQ ID NOs: 8, 12, 16, 20, 24, 26, 30, or 34.

9. A nucleic acid molecule having a sequence encoding a binding molecule as defined in any one of claims 1 to 8.

10. A vector comprising a nucleic acid sequence as defined in claim 9.

11. A host cell transformed or transfected with the nucleic acid sequence as defined in claim 9 or with the vector as defined in claim 10.

12. A process for the production of a binding molecule according to any one of claims 1 to 8, said process comprising culturing a host cell as defined in claim 11 under conditions allowing the expression of the binding molecule as defined in any one of claims 1 to 8 and recovering the produced binding molecule from the culture.

13. A pharmaceutical composition comprising a binding molecule according to any one of claims 1 to 8, or produced according to the process of claim 12.

14. The binding molecule according to any one of claims 1 to 8, or produced according to a method of claim 12 for use in the prevention, treatment or amelioration of a disease selected from the group consisting of a proliferative disease, an inflammatory disease, an infectious disease and an autoimmune disease.

15. A kit comprising a binding molecule as defined in any one of claims 1 to 8 or produced according to a method of claim 12, a nucleic acid molecule as defined in claim 9, a vector as defined in claim 10, or a host cell as defined in claim 11.
